# EUROPÄISCHE PATENTANMELDUNG

(11) **EP 3 910 399 A1**
(43) Veröffentlichungstag der Anmeldung: **17.11.2021**
(21) Anmeldenummer: 20174034.7
(22) Anmeldetag: 12.05.2020
(51) Int. Cl.: G02B 21/16, G02B 21/36, G01N 15/14, G01N 21/64, G01N 33/569, G06K 9/00, G06K 9/62, G06T 7/00

(54) **VERFAHREN ZUR VIRTUELLEN ANNOTATION VON ZELLEN**

(71) Anmelder: Siemens Healthcare Diagnostics, Inc., Tarrytown, NY 10591 (US)
(72) Erfinder: Engel, Thomas, 73432 Aalen (DE); Marquardt, Gaby, 91353 Hausen (DE); Richter, Lukas, 96114 Hirschaid (DE)
(74) Vertreter: Castorph, Simon Johannes

(57) **Zusammenfassung**

Die Erfindung umfasst ein Verfahren zum Trainieren eines Verfahrens zur automatisierten Erkennung, Identifikation, Klassifizierung und/oder virtuellen Annotation einer Zelle, von Bestandteilen einer Zelle und/oder eines biologischen Präparats, das Verfahren umfassend eine Einfärbung der Zelle, der Bestandteile der Zelle und/oder des biologischen Präparats mittels Durchführung eines Färbeprotokolls, wobei das Färbeprotokoll eine selektive Färbung von vorbestimmten Merkmalen der Zelle und/oder des biologischen Präparats umfasst, wobei bevorzugt die vorbestimmten Merkmale zelluläre und/oder subzelluläre Strukturen umfassen, und wobei die Färbung eine selektiv schaltbare Färbung umfasst, ein Ermitteln von zweidimensionalen und/oder dreidimensionalen Informationen der Zelle und/oder des biologischen Präparats mittels eines ersten Analyzers zum Analysieren einer biologischen Probe, der Analyzer umfassend eine Vorrichtung zur Ermittlung der zweidimensionalen und/oder dreidimensionalen Informationen der Zelle und/oder des biologischen Präparats umfassend einen Farbkontrast, wobei der Farbkontrast sensitiv ist auf die Färbung des Färbeprotokolls und wobei das Ermitteln der Informationen für wenigstens zwei unterschiedliche Schaltzustände der selektiv schaltbaren Färbung erfolgt, und eine Erstellung und Speicherung einer Zuordnung zwischen den mit den unterschiedlichen Schaltzustände der selektiv schaltbaren Färbung ermittelten Informationen.

## Beschreibung

Die Erfindung liegt auf dem Gebiet der automatischen Analyzer und betrifft ein Verfahren zum Trainieren eines Verfahrens zur automatisierten Erkennung, Identifikation, Klassifizierung und/oder virtuellen Annotation einer Zelle, von Bestandteilen einer Zelle und/oder eines biologischen Präparats.

Zur automatisierten Analyse von Zellen werden so genannte "automated cell counters" (Automatisierte Zellzählgeräte) mit zunehmendem Erfolg angewendet. Beispiele hierfür sind das Advia 2120, Sysmex XE-2100, CellaVision DM96 sowie das CellaVision DM1200 Gerät. Diese automatisierten Geräte stellen, abgesehen von ihrer hohen Durchsatzzahl, einige Vorteile bereit, wie beispielsweise hohe Objektivität (keine Variabilität abhängig vom Beobachter), Elimination statistischer Variationen, die üblicherweise mit einer manuellen Zählung verbunden sind (Zählung hoher Zellzahlen), sowie die Bestimmung zahlreicher Parameter, die bei einer manuellen Auszählung nicht verfügbar wären und, wie angesprochen eine effizientere und kosteneffektivere Handhabung. Einige dieser Geräte können 120 bis 150 Patientenproben pro Stunde bearbeiten.

Die technischen Prinzipien der automatischen Einzelzellzählung beruhen meist entweder auf einer Impedanz- (Widerstands) Messung oder auf einem optischen System (Streulicht- bzw. Absorptionsmessung). Weiter sind bildgebende Systeme etabliert, die z.B. Zellen eines Blutausstrichs automatisiert abbilden und bewerten.

Beim Impedanzverfahren erfolgt die Zellzählung sowie deren Größenbestimmung auf Grundlage des Nachweises und der Messung von Veränderungen in der elektrischen Leitfähigkeit (Widerstand), die durch ein Teilchen verursacht werden, das sich durch eine kleine Öffnung hindurchbewegt. Teilchen, wie beispielsweise Blutzellen, sind selbst nicht leitend, werden jedoch in einem elektrisch leitenden Verdünnungsmittel suspendiert. Wenn eine derartige Suspension von Zellen durch eine Öffnung hindurchgeleitet wird, nimmt bei Durchgang einer einzelnen individuellen Zelle die Impedanz (Widerstand) des elektrischen Weges zwischen den beiden Elektroden, die sich auf jeder Seite der Öffnung befinden, vorübergehend zu.

Im Gegensatz zum Impedanzverfahren umfasst das optische Verfahren das Durchleiten eines Laserlichtstrahles oder eines LED-Lichtstrahls durch eine verdünnte Blutprobe, die in einem kontinuierlichen Strom von dem Laserstrahl oder dem LED-Lichtstrahl erfasst wird. Der entsprechende Lichtstrahl kann dabei z.B. mittels eines Lichtwellenleiters zur Durchflusszelle geleitet werden. Jede Zelle, die durch die Erfassungszone der Durchflusszelle hindurchtritt, streut das fokussierte Licht. Das gestreute Licht wird dann durch einen Fotodetektor nachgewiesen und in einen elektrischen Impuls umgewandelt. Die hier erzeugte Anzahl von Impulsen ist direkt zur Zellanzahl proportional, die durch die Erfassungszone in einer speziellen Zeitspanne hindurchtritt.

Bei den optischen Verfahren wird die Lichtstreuung der einzelnen Zelle, die durch die Erfassungszone hindurchtritt, in verschiedenen Winkeln gemessen. Hierdurch wird das Streuverhalten der jeweiligen Zelle für die optische Strahlung erfasst, das Rückschlüsse auf z.B. die Zellstruktur, Form und Reflexionsvermögen erlaubt. Dieses Streuverhalten kann dazu verwendet werden, verschiedene Arten von Blutzellen zu differenzieren und die abgeleiteten Parameter zur Diagnose von Abweichungen der Blutzellen dieser Probe von einer Norm, die z.B. aus einer Vielzahl von als normal klassifizierten Referenzproben gewonnen wurde, zu verwenden.

Bei der automatisierten Bewertung von Zellen in Blutausstrichen arbeiten die heutigen Analysatoren mit Mikroskopen mit einer hohen numerischen Apertur und mit Immersion zwischen dem Objektträger und dem Objektiv, um eine hohe Auflösung erreichen zu können. Dadurch ergibt sich jedoch eine vergleichsweise geringe Tiefenschärfe, die deutlich geringer ist als die Dicke der Zellen senkrecht zur Fläche des Objektträgers mit dem Blutausstrich. Entsprechend kann mit einer zweidimensionalen Abbildung in nur einer Fokuseinstellung nicht die gesamte Tiefeninformation der Zelle scharf abgebildet werden.

Häufig kommt es daher zu unklar klassifizierten Blutzellen, die Fachpersonal, z.B. ein Laborarzt, manuell nachklassifizieren muss. Dazu wird der Objektträger mit dem Blutausstrich erneut unter ein Mikroskop gelegt, die entsprechende Zelle aufwändig gesucht und dann von dem Laborarzt inspiziert. Zur sicheren Klassifizierung fokussiert dabei der Laborarzt meist auch durch die Zelle hindurch, um die Struktur der Zelle entlang der Fokusrichtung besser erkennen und bewerten zu können.

WO 2007/044725 A2, Bahram J., et al.: "Three-dimensional identification of biological microorganism using integral imaging" in Optics Express, Bd. 14, Nr. 25, Seiten 12096 - 566, Kim J. et al.: "A single-shot 2D/3D simultaneous imaging microscope based on light field microscopy", in Visual Communications and image processing, Bd. 9655, Seiten 96551O1 - 96551O4 und WO 2010/121637 A1 beschreiben optische Abbildungsvorrichtungen.

Die momentan gebräuchlichen Messgeräte in der Hämatologie umfassen bezüglich des optischen Systems ein Mikroskop mit etwa 100-facher Vergrößerung mit effektiven lateralen Auflösungen eines Sensorelements auf der Objektebene von 100 nm = 0,1 µm. Die gebräuchlichen Kameras haben Pixelzahlen von max. 0,3 bis 1 Millionen Pixeln. Dadurch ist das Gesichtsfeld im Objekt nur wenige 100 µm groß. Um den gefärbten Ausstrich einer Blutprobe, der einige mm breit und mehrere 10 mm lang sein kann, aufnehmen und analysieren zu können, muss dann die Fläche des zu analysierenden Bereichs des Ausstriches mit einer Verschiebeeinheit in einem Mäanderscanverfahren abgetastet werden. Um den Prozess etwas zu beschleunigen wird ein erster flächenhafter Scan mit geringer Vergrößerung, z.B. 10-fach mit entsprechend 10-fach größerem Gesichtsfeld durchgeführt und nach einer ersten Bildauswertung zum Auffinden der zu vermessenden Zellen werden dann nur die Regions of Interest (RoI) mit den Zellen mit der höheren Vergrößerung anschließend gezielt angefahren. Das bedeutet, dass eine vollständige flächenhafte Analyse der Probe mit der hohen Vergrößerung nicht erfolgt. Um die Zellen hinreichend gut sichtbar zu machen, um sie mit der hochauflösenden optischen Mikroskopie analysieren zu können, werden die Blutausstriche in einem vorgelagerten Schritt angefärbt. Es haben sich mehrere Färbeprotokolle weltweit etabliert, die sich global gesehen teilweise von Region zu Region unterscheiden. Somit ist die Vergleichbarkeit der Analyse von Blutausstrichen regional begrenzt, da nur Bilder von Zellen gut miteinander verglichen werden können, die nach demselben Protokoll angefärbt worden sind.

Weiter ist die Färbung der Zellen der Blutausstriche sehr zeitaufwändig und in ihrer Reproduzierbarkeit begrenzt, da z.B. Reagenzien sich durch Alterungsprozesse verändern oder unterschiedliche Labore nach unterschiedlichen Protokollen färben. Ähnliche oder gleiche Probleme ergeben sich bezüglich der Färbung und Untersuchung von anderen biologischen Präparate, wie z.B. Gewebeproben oder Dünnschnitten von Gewebeproben. Ist eine Probe einmal angefärbt, so ist diese Färbung dauerhaft in der Probe vorhanden und der Färbeprozess kann in der Regel nicht mehr ohne Weiteres rückgängig gemacht werden.

Es gibt prinzipiell verschiedene Versuche und Ansätze, biologische oder medizinische Proben von Zellen und/oder Geweben mit optischen Abbildungssystemen aufzunehmen und die technischen Bilder danach auf der Basis von Strukturerkennung und/oder Segmentierung von Bereichen der Probe und/oder des erhaltenen optischen Kontrastes digital einzufärben. Gemeinsam ist diesen Ansätzen, dass versucht wurde, die Färbung auf Basis der Bildstruktur zusammen mit einer Strukturinformation und/oder Kontrastinformation anzufärben. Beispielhaft wird zum Einfärben sowohl die über Methoden des Computerlernens mit neuronalen Netzen (deep CNNs, CNNs) oder künstlicher Intelligenz (KI, AI) eine Struktur in Bilddaten erkannt, entsprechend der Struktur bzw. des Strukturelements dann eine Färbung für diesen Bereich ausgewählt und die Farbsättigung bzw. Intensität dann z.B. anhand der interferometrisch bestimmten optischen Pfadlänge bzw. optischen Dichte bestimmt.

Beim Training solcher automatisierter, computerbasierter Systeme zur Diagnostik beziehungsweise zur Unterscheidung von z.B. Zellen, Zellkern, Zytoplasma, Zytoskelett oder anderen Zellstrukturen oder Organellen ist es wichtig, möglichst präzise Referenzdaten, die teilweise auch als Ground Truth Daten bezeichnet werden, in einer ausreichenden Menge und Qualität zur Verfügung zu haben. Hierzu ist es bisher notwendig, dass die Bilder bzw. die entsprechenden Zellen oder Zellstrukturen händisch von entsprechenden Experten, z.B. erfahrenen Labormedizinern, etc. einzeln annotiert werden. Dies ist sehr zeitaufwändig und teilweise auch fehleranfällig, da die jeweilige Einteilung oder Zuordnung auch subjektive Momente nicht immer völlig ausschließen kann. Dies führt letztlich zu weniger zuverlässigen Ergebnissen in der späteren automatischen Zuordnung durch die Algorithmen, da diese teilweise nur unzureichend trainiert sind, und somit zu weniger zuverlässigen Ergebnissen bei der digitalen Anfärbung der Zellen oder Zellbestandteilen.

**Eine der Erfindung zugrundeliegende Aufgabe ist somit das Bereitstellen eines Verfahrens zur automatisierten Erkennung, Identifikation, Klassifizierung und/oder virtuellen Annotation einer Zelle, von Bestandteilen einer Zelle und/oder eines biologischen Präparats, das eine zuverlässigere Erkennung, Identifikation, Klassifizierung und/oder virtuellen Annotation der Zelle, der Bestandteile der Zelle und/oder des biologischen Präparats ermöglicht.**

Die Erfindung geht dabei von der Idee aus, dass sich eine eindeutige Markierung von Zellbestandteilen bzw. Organellen mit selektiv anregbaren Markern erreichen lassen. Die jeweilige von dem betreffenden Farbstoff markierte Zellstruktur wird dabei über das emittierte Licht eindeutig markiert und in der optischen Abbildung einzeln erkennbar. So können insbesondere auch sich im mikroskopischen Bild überlagernde Strukturen durch sequenzielle Anregung getrennt dargestellt werden. Weiter kann auch nach erfolgter Färbung der Probe bei Bedarf die Färbung durch entsprechendes Ausschalten effektiv wieder rückgängig gemacht werden und kann insofern auch als reversibel betrachtet werden.

Die Erfindung betrifft dabei u.a. ein Verfahren zum Anlernen von einem Algorithmus zur automatischen Erkennung von Zellen beziehungsweise Zellbestandteilen nur auf Basis eines Bildes und/oder eines aggregierten multimodalen Bildes, das nur mit einem oder mehreren technischen Kontrasten aufgenommen wurde, bereitgestellt. Weiter betrifft die Erfindung u.a. ein Verfahren zum funktionalen Annotieren von Zellstrukturen für das Anlernen der computergestützten Identifikation und Klassifizierung von Zellen und Zellstrukturen mittels selektiv anregbarer Farbstoffe, die die unterschiedlichen Bestandteile der Zellen oder Probe in möglichst eindeutiger Weise kennzeichnen.

Die der Erfindung zugrundeliegende Aufgabe wird insbesondere von einem erfindungsgemäßen Verfahren gemäß der unabhängigen Patentansprüche gelöst. Vorteilhafte Weiterbildungen der Erfindung sind insbesondere auch durch die Unteransprüche gegeben.

Der Gegenstand der Erfindung umfasst ein erfindungsgemäßes Verfahren zum Trainieren eines Verfahrens zur automatisierten Erkennung, Identifikation, Klassifizierung und/oder virtuellen Annotation einer Zelle, von Bestandteilen einer Zelle und/oder eines biologischen Präparats, das Verfahren umfassend die Schritte
a) Einfärbung der Zelle, der Bestandteile der Zelle und/oder des biologischen Präparats mittels Durchführung eines Färbeprotokolls, wobei das Färbeprotokoll eine selektive Färbung von vorbestimmten Merkmalen der Zelle und/oder des biologischen Präparats umfasst, wobei bevorzugt die vorbestimmten Merkmale zelluläre und/oder sub-zelluläre Strukturen umfassen, und wobei die Färbung eine selektiv schaltbare Färbung umfasst,
b) Ermitteln von zweidimensionalen und/oder dreidimensionalen Informationen der Zelle und/oder des biologischen Präparats mittels eines ersten Analyzers zum Analysieren einer biologischen Probe, der Analyzer umfassend eine Vorrichtung zur Ermittlung der zweidimensionalen und/oder dreidimensionalen Informationen der Zelle und/oder des biologischen Präparats umfassend einen Farbkontrast, wobei der Farbkontrast sensitiv ist auf die Färbung des Färbeprotokolls und wobei das Ermitteln der Informationen für wenigstens zwei unterschiedliche Schaltzustände der selektiv schaltbaren Färbung erfolgt,
c) Erstellung und Speicherung einer Zuordnung zwischen den mit den unterschiedlichen Schaltzustände der selektiv schaltbaren Färbung ermittelten Informationen.

Das erfindungsgemäße Verfahren weist u.a. den Vorteil auf, dass die Ermittlung der Referenzinformation, auch teilweise als "Ground Truth" bezeichnet, automatisiert erfolgen kann und auf eine manuelle Annotation verzichtet werden kann. Damit wird dieser Prozess erfindungsgemäß weniger Fehleranfällig und die dadurch maximal erreichbare Prozesssicherheit in der nachfolgenden automatischen Klassifizierung entsprechend erhöht.

Die Bestimmung der Ground Truth über selektiv schaltbare Färbung, insbesondere nachdem die Färbung durchgeführt wurde, ist vollständig automatisiert auswertbar, was eine vollständige Automatisierung der Prozesse erlaubt. Zudem wird eine hohe Auflösung ermöglicht, die im Allgemeinen grundsätzlich z.B. nur über die Pixelauflösung des verwendeten Aufnahmesystems begrenzt ist. Man braucht kein wissenschaftlich hoch geschultes Personal mehr für die Annotation, sondern nur noch einen Bediener für das Gerät und die Ausführung der Färbung mit der selektiv schaltbaren Färbung. Aufgrund der möglichen Automatisierung kann die Bestimmung der Ground Truth somit viel schneller als mit Menschen und zudem mit geringerer Fehleranfälligkeit bzw. höherer Reproduzierbarkeit durchgeführt werden.

Weiter können insbesondere auch sich beim Ermitteln der zweidimensionalen und/oder dreidimensionalen Informationen, z.B. im mikroskopischen Bild, überlagernde Strukturen durch sequentielle Anregung getrennt dargestellt werden und die entsprechende Information in der Zuordnung zwischen den mit den unterschiedlichen Schaltzustände der selektiv schaltbaren Färbung ermittelten Informationen somit berücksichtigt werden. Somit können in der nachfolgenden automatischen Klassifizierung solche Überlagerungen ebenfalls aufgelöst und dargestellt werden.

Die erfindungsgemäße Bestimmung der Ground Truth mittels der selektiv schaltbaren Färbung, z.B. selektiv anregbarer Farbstoffe, hat weiter den Vorteil, dass die über den jeweiligen Farbstoff markierbaren Bestandteile der Probe vollständig markiert werden, da die Einfärbung zumeist nasschemisch erfolgt. Somit kann davon ausgegangen werden, dass die Annotation vollständiger und präziser erfolgt als von Menschen ausführbar an Bildern, zumal diese dann auch gefärbt sein müssten.

Bevorzugt umfasst die Annotation eine Klassifizierung der Zelle, der Bestandteile der Zelle und/oder des biologischen Präparats.

Bevorzugt umfasst die Klassifizierung eine Unterscheidung von verschiedenen Zelltypen.

Bevorzugt ist das biologische Präparat ein medizinisches Präparat.

Bevorzugt umfasst eine virtuelle Annotation eine virtuelle Anfärbung der Zelle, von Bestandteilen der Zelle und/oder des biologischen Präparats. Eine virtuelle Anfärbung wird auch teilweise als eine digitale Anfärbung bezeichnet.

Subzellulare Strukturen umfassen bevorzug Zytoskelett, Zytoplasma und/oder Organellen, wie z.B. Zellkern und/oder Golgi-Apparat.

In einer bevorzugten Ausgestaltung des Verfahrens erfolgt vor Einfärbung der Zelle, der Bestandteile der Zelle und/oder des biologischen Präparats in Schritt a) eine Ermittlung von zweidimensionalen und/oder dreidimensionalen Informationen der Zelle, der Bestandteile der Zelle und/oder des biologischen Präparats ohne Färbung mittels eines zweiten Analyzers zum Analysieren einer biologischen Probe, der Analyzer umfassend eine Vorrichtung zur Ermittlung der zweidimensionalen und/oder dreidimensionalen Informationen der Zelle, der Bestandteile der Zelle und/oder des biologischen Präparats mit zellulären und/oder sub-zellulären Strukturen, die bevorzugt technische Kontrastierungsverfahren zur Gewinnung der zweidimensionalen und/oder dreidimensionalen Information anwendet, und wobei weiter eine Erstellung und Speicherung einer Zuordnung zwischen den vor der Anfärbung und nach der Anfärbung ermittelten zweidimensionalen und/oder dreidimensionalen Informationen der Zelle, der Bestandteile der Zelle und/oder des biologischen Präparats erfolgt. Dies hat den Vorteil, dass die Anfärbung nicht schon unmittelbar bei der Probenpräparation erfolgt, sondern nach einer ersten Vermessung der Probe mit den technischen Kontrasten, um eine Querwirkung der eingebrachten Färbung bzw. Farbstoffe auf das ursprüngliche Kontrastierungsverhalten der Probe für die technischen Kontraste zu vermeiden.

In einer bevorzugten Ausgestaltung des Verfahrens handelt es sich bei dem ersten Analyzer und dem zweiten Analyzer um das gleiche Gerät, wobei der Analyzer bevorzugt jeweils in unterschiedlichen Messkonfigurationen betrieben wird. Dies hat den Vorteil, dass das Verfahren auf einem einzigen Analyzer durchgeführt werden kann. Dies vereinfacht u.a. auch die möglichst vollständige Automatisierung der Prozesse.

Die unterschiedlichen Messkonfigurationen umfassen bevorzugt z.B. eine Hardware-Umschaltung zwischen einem Differentialinterferenzkontrast (DIC) und einem Fluoreszenzmodus, und/oder eine Software-Umschaltung zwischen Farbkanälen bei einer Fluoreszenzmessung.

In einer bevorzugten Ausgestaltung des Verfahrens hat die Färbung in einem der Schaltzustände keinen oder nur einen möglichst geringen Einfluss auf das Ermitteln von zweidimensionalen und/oder dreidimensionalen Informationen der Zelle, der Bestandteile der Zelle und/oder des biologischen Präparats und wobei die Zelle, die Bestandteile der Zelle und/oder das biologische Präparat in diesem Schaltzustand besonders bevorzugt ungefärbt erscheint.

In einer bevorzugten Ausgestaltung des Verfahrens umfasst das Verfahren zusätzlich eine weitere Einfärbung der Zelle, der Bestandteile der Zelle und/oder des biologischen Präparats mittels Durchführung eines weiteren Färbeprotokolls, wobei das weitere Färbeprotokoll eine statische Anfärbung der Zelle, der Bestandteile der Zelle und/oder des biologischen Präparats umfasst und wobei die weitere Einfärbung bevorzugt nach Durchführung der Schritte a), b) und c) erfolgt.

Bevorzugt handelt es sich bei der statischen Anfärbung dabei um eine irreversible Anfärbung.

Bevorzugt handelt es sich bei dem weitern Färbeprotokoll z.B. um das Färbeprotokoll May-Grünwald-Giemsa, Modified-Wright Färbung, Wright-Gimsa Färbung und/oder Romanowsky Färbung.

In einer bevorzugten Ausgestaltung des Verfahrens umfasst die Färbung mindestens zwei verschiedene, unabhängig voneinander selektiv schaltbare Färbungen. Dies hat den Vorteil, dass im Vergleich zu einer statischen Färbung auch räumlich übereinanderliegende Bereiche einer Zelle jeweils selektiv erfasst werden können und so besser oder gar überhaupt erst differenzierbar werden.
In einer bevorzugten Ausgestaltung des Verfahrens ist die Färbung mittels eines externen Stimulus selektiv ein und/oder ausschaltbar, wobei der externe Stimulus bevorzugt eine optische, thermische, elektrische und/oder magnetische Einwirkung auf die Färbung und/oder die Probe umfasst.

Vorteilhafterweise sind die selektiv anregbaren Farbstoffe prinzipiell in ihrer biologischen oder chemischen Bindung auf mehrere oder bevorzugt einen in der optischen Abbildung kontrastierbaren und damit differenzierbaren Zell- oder Gewebebestandteil selektiv, wie z.B. das Zytoskelett, Golgi, Nuklei, Nukleoli, Zytoplasma oder auch über z.B. Gewebetypen und Gewebebestandteile mit stofflichen oder zellulären Eigenschaften wie DNA und/oder bestimmte Gene oder Gensequenzen ankoppeln und entsprechend ausgewählt werden. Wie aus den Methoden der Fluoreszenzmikroskopie, z.B. Facs oder FRET bekannt, hat z.B. ein Mikroskop dann technisch so ausgerüstet zu sein, dass die verwendeten Farbstoffe selektiv angeregt werden können und/oder das von der Probe emittierte Licht entsprechend von dem Licht der Beleuchtung oder selektiv gefiltert aufgenommen werden kann.

In einer bevorzugten Ausgestaltung des Verfahrens umfasst die Färbung einen oder mehrere Farbstoffe.

In einer bevorzugten Ausgestaltung des Verfahrens umfasst die Färbung schaltbare Markierungsmoleküle.

In einer bevorzugten Ausgestaltung des Verfahrens umfassen die schaltbaren Markierungsmoleküle eine oder mehrere Fluorophore. Dabei werden, die Fluorophore, ähnlich wie z.B. auch in der computergestützten Mikroskopie, eingesetzt, um über eine gezielte Anregung eines Fluoreszenzfarbstoffs spezifische Bereiche der Zelle oder Zellstruktur sichtbar zu machen. Wenn unterschiedliche Fluorophore zum Einsatz kommen, können diese z.B. mit unterschiedlichen Wellenlängen angeregt werden und so selektiv zum Leuchten gebracht werden. Im Nachhinein werden dann die mit unterschiedlichen Beleuchtungswellenlängen und damit unterschiedlichen Fluorophoren aufgenommen Bilder zu einem Gesamtbild fusioniert. Die unterschiedlichen Fluorophore werden vorteilhafterweise so ausgewählt, dass jeder der Farbstoffe entweder selbst oder über eine biologisch aktive Gruppe, die am Farbstoff angebunden ist, an unterschiedliche Bereiche bzw. Zellbestandteile oder auch DNA-Gruppen bzw. Substanzen anlagert und so dann über den Fluorophor diese Bereiche dann selektiv sichtbar werden.

Dies ermöglicht z.B. auch die Anwendung von modernen Methoden der biologischen bzw. medizinischen Bildgebung, die u.a. als Fluoreszenzmikroskopie, Facs (Fluorescence-activated cell scanning) oder FRET (Fluorescence Resonance Energy Transfer) bekannt sind. Dabei können teilweise die Auflösungsgrenzen der Mikroskopie unter das klassischer Auflösungslimit nach Airy für die Optik allein verschoben werden. Solche Methoden können dabei grundsätzlich sowohl bildgebend als auch für rein quantitative Analysen eingesetzt werden, wie z.B. bei Facs. Mit den Methoden können z.B. über 3D Scans auch volumetrische 3D Bilddatensätze gewonnen werden.

Alternativ zu den Fluorophoren oder auch optional zusätzlich dazu können auch Substanzen mit anderen optisch kontrastierenden Eigenschaften verwendet werden, wobei es sich bei den optisch kontrastierenden Eigenschaften z.B. um eine spektrale Absorption, eine Polarisationswirkung (z.B. Drehung der Polarisationsebene), eine Depolarisation, eine Chiralität (z.B. zirkulare Polarisation) und/oder eine Doppelbrechung handelt, die dann über jeweilige entsprechende Kontrastierungsverfahren abbildbar und somit in Bildern darstellbar sind.

Tritt z.B. ein bestimmter Umweltstimulus auf, lösen die entsprechenden Indikatoren eine Reaktion aus, die ein farbiges fluoreszierendes Signal erzeugt. Typische Stimuli sind chemische Reaktionen oder Veränderungen von Temperatur, Licht und Druck. Das Indikatorsystem kann auf viele solcher Veränderungen hin farbige und auch fluoreszierende Signalstoffe erzeugen, welche Rückschlüsse auf die jeweiligen Stimuli zulassen. So kann in der Biologie beispielsweise auch das Aktivieren eines bestimmten Enzyms oder das Binden eines Antikörpers erkannt und angezeigt werden.

In einer bevorzugten Ausgestaltung des Verfahrens sind die markierten Bestandteile der Zelle und/oder des medizinischen Präparats Organellen.

In einer bevorzugten Ausgestaltung des Verfahrens stellen die Markierung eine eindeutige Markierung der vorbestimmten Merkmale dar.

In einer bevorzugten Ausgestaltung des Verfahrens handelt es sich bei den ermittelte zweidimensionalen und/oder dreidimensionale Information um zweidimensionale und/oder dreidimensionale Bildinformationen, bevorzugt um optische Bildinformationen im Realraum.

In einer bevorzugten Ausgestaltung des Verfahrens sind die markierten Bestandteile mittels der Bildinformationen selektiv erkennbar.

In einer bevorzugten Ausgestaltung des Verfahrens sind sich räumlich in der Bildinformation überlagernde Merkmale, die unterschiedlich markiert sind, getrennt darstellbar.

In einer bevorzugten Ausgestaltung des Verfahrens erfolgt die getrennte Darstellbarkeit aufgrund unterschiedlicher Stimuli und/oder der emittierten Lichtwellenlängen der Markierungsmoleküle.

In einer bevorzugten Ausgestaltung des Verfahrens erfolgt die selektive Schaltbarkeit mittels unterschiedlicher Anregungs- und/oder Detektionswellenlängen, wobei die Schaltbarkeit bevorzugt optisch erfolgt.

Bevorzugt umfasst dabei ein Detektor einen Multispektral und/oder Hyperspektraldetektor. In einer bevorzugten Ausgestaltung des Verfahrens werden die Bildinformation der markierten Zellen, der Bestandteile der Zellen und/oder biologischen Präparate als Referenz für Bildinformationen mit technischem Kontrast ohne vorherige Anfärbung verwendet, bevorzugt zur Identifikation von Bildinformationen, die geeignet sind zur Unterscheidung verschiedener Zelltypen und/oder anderer morphologischer Eigenschaften der Probe auf Grundlage von Bildinformationen mit technischem Kontrast ohne vorherige Anfärbung der Zellen, der Bestandteile der Zellen und/oder der biologischen Präparate.

Es werden also bevorzugt die Bildinformation der markierten Zellen und/oder medizinischen Präparate als Referenz für Bildinformationen mit technischem Kontrast ohne vorherige Anfärbung verwendet.

Ein weiterer Gegenstand der Erfindung ist ein Verfahren zur virtuellen Annotation einer Zelle, von Bestandteilen einer Zelle und/oder eines biologischen Präparats, das Verfahren umfassend die Schritte
aa) Ermitteln von zweidimensionalen und/oder dreidimensionalen Informationen der Zelle, von Bestandteilen der Zelle und/oder des biologischen Präparats mittels eines Analyzers zum Analysieren einer biologischen Probe, der Analyzer umfassend eine Vorrichtung zur Ermittlung der zweidimensionalen und/oder dreidimensionalen Informationen der Zelle, der Bestandteile der Zelle und/oder des biologischen Präparats,
bb) virtuelle Annotation der Zelle, der Bestandteile der Zelle und/oder des biologischen Präparats entsprechend einer vorbestimmten Zuordnung zwischen den zweidimensionalen und/ oder dreidimensionalen Informationen der Zelle, der Bestandteile der Zelle und/oder des biologischen Präparats und einer Färbung einer entsprechenden Zelle, von Bestandteile einer Zelle und/oder eines biologischen Präparats und/oder zellulärer und/oder sub-zellulären Strukturen der Zelle, der Bestandteile der Zelle und/oder des biologischen Präparats mittels eines Färbeprotokolls,
cc) Repräsentierung und/oder Speicherung einer Darstellung der virtuell annotierten Zelle, der Bestandteile der Zelle und/oder des biologischen Präparats,
wobei die Zuordnung in Schritt bb) mittels eines erfindungsgemäßen Verfahrens zum Trainieren eines Verfahrens zur automatisierten Erkennung, Identifikation, Klassifizierung und/oder virtuellen Annotation einer Zelle, von Bestandteilen einer Zelle und/oder eines biologischen Präparats bestimmt wurde.

Bevorzugt umfasst dabei die virtuelle Annotation eine virtuelle bzw. digitale Anfärbung der Zelle, von Bestandteilen der Zelle und/oder des biologischen Präparats.

Dies ermöglicht z.B. in einen automatisierten Zellanalysator, der z.B. in der Hämatologie eingesetzt wird, Zellproben ohne vorherige Färbung zu untersuchen. Dazu werden mit technischen Kontrastierungen, wie sie z.B. aus der Mikroskopie bekannt sind, zumindest Bereiche der Proben unmittelbar untersucht bzw. abgebildet und anschließend die Zellstrukturen bzw. Regionen z.B. in einem 2D oder 3D Bild erkannt. Diese erkannten Bereiche können dann z.B. für eine Bewertung der jeweiligen Probe bzw. auch zur Befundung von Krankheitsbildern oder Pathologien verwendet werden.

Das erfindungsgemäße Verfahren weist u.a. den Vorteil auf, dass eine z.B. chemische Vorbehandlung der Zelle weitest möglichst unterbleiben kann, bevor die Bildaufnahme und damit die Analyse stattfindet, um die Zelle in einem möglichst ursprünglichen und wenig oder überhaupt nicht veränderten Zustand untersuchen zu können. Es kann also auf die zeitaufwändige Färbung medizinischer bzw. biologischer Präparate, wie z.B. Blutausstriche, Gewebeproben oder Dünnschnitten von Gewebeproben verzichtet werden, die bisher typischerweise etwa 15 Minuten in Anspruch nehmen kann. Dies hat weiter z.B. auch Vorteile, da eine herkömmliche Färbung in ihrer Reproduzierbarkeit grundsätzlich begrenzt ist, z.B. aufgrund von Alterungsprozessen von verwendeten Reagenzien bzw. Chemikalien oder da unterschiedliche Labore nach unterschiedlichen Protokollen färben. Da keine chemischen Substanzen mehr verbraucht werden reduziert sich der Abfall an chemischen Substanzen. Weiter wird der gesamte Analyseprozess vereinfacht, was auch das Risiko von z.B. Querkontaminationen von unterschiedlichen Proben reduziert. Ein weiterer Vorteil ist, dass ein Wechsel zwischen unterschiedlichen Färbemodellen möglich ist, um z.B. spezifischer für bestimmte Pathologien zu sein.

Ein besonderer Vorteil des erfindungsgemäßen Verfahrens ist es dabei, dass aufgrund technischer Kontraste mittels Mikroskopie erzeugte Bilder nachträglich so eingefärbt werden können, wie es im realen Färbeprozess auch stattgefunden hätte und z.B. dem Biologen oder Mediziner weiterhin ein Bild der Probe zu liefern, so wie er oder sie es aus den bisherigen und etablierten Verfahren gewöhnt ist. Dabei werden insbesondere auch in der Beobachtungsrichtung übereinander liegende Zellbestandteile in der Probe getrennt darstellbar bzw. dargestellt.

Ein weiterer Gegenstand der Erfindung ist ein Verfahren zur automatisierten Erkennung, Identifikation und/oder Klassifizierung einer Zelle, von Bestandteilen einer Zelle und/oder eines biologischen Präparats, das Verfahren umfassend die Schritte
aaa) Ermitteln von zweidimensionalen und/oder dreidimensionalen Informationen der Zelle, von Bestandteilen der Zelle und/oder des biologischen Präparats mittels eines Analyzers zum Analysieren einer biologischen Probe, der Analyzer umfassend eine Vorrichtung zur Ermittlung der zweidimensionalen und/oder dreidimensionalen Informationen der Zelle, der Bestandteile der Zelle und/oder des biologischen Präparats,
bbb) automatisierte Erkennung, Identifikation und/oder Klassifizierung der Zelle, der Bestandteile der Zelle und/oder des biologischen Präparats aufgrund einer vorbestimmten Zuordnung zwischen den zweidimensionalen und/oder dreidimensionalen Informationen der Zelle, der Bestandteile der Zelle und/oder des biologischen Präparats und einer Färbung einer entsprechenden Zelle, der Bestandteile einer Zelle und/oder eines biologischen Präparats und/oder zellulärer und/oder sub-zellulären Strukturen der Zelle, der Bestandteile der Zelle und/oder des biologischen Präparats mittels eines Färbeprotokolls, wobei die Zuordnung mittels eines erfindungsgemäßen Verfahrens zum Trainieren eines Verfahrens zur automatisierten Erkennung, Identifikation, Klassifizierung und/oder virtuellen Annotation einer Zelle, von Bestandteilen einer Zelle und/oder eines biologischen Präparats bestimmt wurde.

Ein weiterer Gegenstand der Erfindung ist ein Analyzer umfassend eine Steuervorrichtung und/oder eine Auswertevorrichtung, wobei die Steuervorrichtung und/oder die Auswertevorrichtung so konfiguriert sind, dass der Analyzer ein erfindungsgemäßes Verfahren oder mehrere erfindungsgemäße Verfahren durchführen kann.

In einer weiteren vorteilhaften Ausgestaltungen der erfindungsgemäßen Verfahren umfassen die ermitteltem zweidimensionalen und/oder dreidimensionale Information Informationen der Probe auf zellulären und/oder sub-zellulären Größenordnungen.

Es werden erfindungsgemäß vorteilhafterweise echte 3D Bildinformationen aus der Probe auf zellulärem und sub-zellulärem Level verwendet, um die Farben bzw. die lokale Färbung der Probe anhand von technisch kontrastierten Strukturinformation von den Teilbereichen der Zelle oder des Gewebes zu bestimmen. Es erfolgt dabei eine Unterscheidung von Zellbereichen in allen drei Dimensionen der Bildaufnahme auf mikroskopischem Level und auch in den sub-zellulären Strukturen.

Die Ausgabe des Bildes erfolgt z.B. für eine jeweils gewählte Fokusposition vorteilsweise als 2D-Bild aufgrund der dreidimensional erfassten Daten. Durch eine Änderung der Fokusposition kann z.B. der Arzt oder Biologe als Beobachter dann wie vom herkömmlichen Mikroskop gewohnt durch die Probe hindurch fokussieren. Es ist dabei auch möglich, aufgrund der dreidimensionalen Daten, die Dicke der Probenschicht und die Fokuslage im Nachhinein anzupassen und auszuwählen und so dann z.B. dem Arzt oder Biologen die Möglichkeit zu geben, Teilbereich noch gezielter anschauen zu können, als es bisher möglich war.

Da die unterschiedlichen Zelltypen bzw. Strukturen in Zellen vorteilhafterweise unterschiedlich eingefärbt werden, ist es auch möglich, dass gewisse Strukturen vom Beobachter im mikroskopischen Bild ausgewählt werden, die nicht dargestellt werden sollen. So können nun z.B. auch räumliche Strukturen einzelner Probenbestandteile gezielt und isoliert dargestellt, betrachtet und bewertet werden.

Die Einfärbung der einzelnen Zellbestandteile erfolgt vorteilhafterweise z.B. in dem ein Anwender eine entsprechende Farbtabelle auswählt, in der bestimmten Zellstrukturen oder Zellen dann bestimmte Farben zugeordnet werden. Diese Farbtabelle kann dann beispielsweise einem der bekannten Färbeprotokolle entsprechen. Die jeweilige Farbsättigung wird dann basierend auf der 2D und/oder 3D Information bestimmt.

In einer weiteren bevorzugten Ausführung des erfindungsgemäßen Verfahrens umfasst das Verfahren eine Segmentierung der ermittelten zweidimensionalen und/oder dreidimensionalen Informationen zur Erkennung zellulärer und/oder sub-zellulären Strukturen der Probe.

In einer weiteren bevorzugten Ausführung des erfindungsgemäßen Verfahrens handelt es sich bei den ermittelte zweidimensionalen und/oder dreidimensionalen Informationen um zweidimensionale und/oder dreidimensionale Bildinformationen, bevorzugt um Bildinformationen im Realraum.

In einer weiteren bevorzugten Ausführung des erfindungsgemäßen Verfahrens umfasst die ermittelte dreidimensionale Information holographische Informationen.

In einer weiteren bevorzugten Ausführung des erfindungsgemäßen Verfahrens umfasst der Analyzer zur Ermittlung der zweidimensionalen und/oder dreidimensionalen Information in ein optisches Mikroskop zum Abbilden eines Lichtfelds in einem Objektbereich zur Abbildung der Probe umfasst, das Mikroskop umfassend eine Lichtquelle zum Beleuchten der Probe und ein Objektiv umfassend eine Sammellinse zum Sammeln und Fokussieren von Lichtstrahlen ausgehend von der beleuchteten Probe sowie ein digitales Aufzeichnungsgerät zum Aufzeichnen der Lichtstrahlen, wobei an dem Mikroskop eine Lichtfeldkamera zum Aufnehmen des im Mikroskop abgebildeten Lichtfelds aus dem Objektbereich vorgesehen ist, wobei bevorzugt die Lichtfeldkamera das digitale Aufzeichnungsgerät umfasst.

Bevorzugt handelt es sich bei dem Analysator um einen automatischen Analysator, besonders bevorzugt um einen teilautomatischen oder vollautomatischen Analysator. Besonders bevorzugt handelt es sich bei dem Analysator um einen automatischen Hämatologie-Analyzer und wobei es sich bei der Zelle bevorzugt um eine Blutzelle handelt.

Bevorzugt umfasst das Mikroskop die Lichtfeldkamera. Bevorzugt umfasst die Lichtfeldkamera das digitale Aufzeichnungsgerät, das zum Aufnehmen des im Mikroskop abgebildeten Lichtfelds ausgebildet ist. Bevorzugt umfasst das digitale Aufzeichnungsgerät einen charge-coupled device (CCD) Chip oder mehrere CCD Chips. Besonders bevorzugt basiert das digitale Aufzeichnungsgerät auf einer Complementary Metal-Oxide-Semiconductor (CMOS) Technik und/oder umfasst einen CMOS Chip.

Bevorzugt erfolgt die Verbringung der Probe innerhalb des automatischen Analyzers dabei vollautomatisch, z.B. durch entsprechende Durchflusssysteme und/oder Verfahrung von Probenträgern innerhalb des Analyzers, bevorzugt mittels entsprechend gesteuerten Aktuatoren oder Robotersystemen.

Bevorzugt umfasst die Lichtfeldkamera ein Mikrolinsenarray mit Linsen unterschiedlicher Brennweite, wobei das Mikrolinsenarray ein Zwischenbild des im Mikroskop abgebildeten Lichtfelds auf das digitale Aufzeichnungsgerät abbilden kann.

Das Mikrolinsenarray ist dabei mindestens eine Brennweite von dem Zwischenbild entfernt und es erfolgt eine reelle Abbildung. Es entstehen also erfindungsgemäß keine Aperturbilder, sondern reale, kleine Bildausschnitte im Sinne kleiner Teilbilder vom Objekt bzw. der Probe. Die Bildinformation wird dann durch die Optik zurück projiziert, bis sich die Strahlen von sich entsprechenden Bildpunkten aus unterschiedlichen Teilbildern treffen. Es werden also vorliegend keine Richtungsbilder, sondern kleine Objektbilder aufgenommen.

Vorteilhafterweise basiert der Analyzer auf einem optischen Mikroskop, das beispielweise mit Vorrichtungen für Differential Interferenzkontrast ausgestattet ist. Solche Mikroskope können dabei in Bezug auf die Aufspaltung auf die Anforderungen der Hämatologie angepasst werden. Diese Anpassung erfolgt im Wesentlichen durch eine gezielte Wahl des Strahlversatzes auf dem Strahlweg durch das Messobjekt. Bei dem Messobjekt handelt es sich beispielsweise um eine ausgestrichene Blutprobe.

Der Strahlversatz wird durch die Dicke der DIC-Prismen im Beleuchtungsmodul und im Analysatormodul bestimmt. Hierbei muss die optische Dicke der beiden Prismen und die Richtung des Strahlversatzes zueinander abgestimmt sein, damit das Analysatormodul den Strahlversatz aus dem Beleuchtungsmodul wieder vollständig umkehren und damit kompensieren kann. Aufgrund unterschiedlicher Abbildungsmaßstäbe können so die physikalischen Dicken der Prismen voneinander abweichen. Bei Mikroskopen kann es für ein Objektiv mehrere Paare von DIC Prismen geben, die unterschiedliche Aufspaltung ermöglichen.

Ferner kann vorteilhafterweise an dem Mikroskop eine Lichtfeldkamera, auch plenoptische Kamera genannt, vorgesehen sein, die das im Mikroskop abgebildete Lichtfeld aus der Objektebene aufnimmt.

Bevorzugt weist die Lichtfeldkamera eine effektive Blendenzahl (working f#) im Bereich von 10 bis 30 auf, besonders bevorzugt beträgt sie 26. Je nach angestrebter Abtastung des Messobjektes mit Sensorelementen wird dazu eine geeignete Vergrößerung gewählt, die die Größenbeziehung vom realen Sensorelement, das im Bereich von typischerweise 1 bis 10 µm liegt und der Abtastung des Objektes, die im Bereich von 0,05 bis 0,5 µm liegt, wobei bevorzugt wegen der hohen Auflösungsanforderungen bei der Messung der Bereich von 0,05 µm bis 0,15 µm ist, berücksichtigt. Damit ergeben sich dann effektive Systemvergrößerungen vom Objekt bis zur Detektionseinrichtung im Bereich von 10-fach bis 100-fach, bevorzugt im Bereich von 20-fach bis 65-fach und besonders bevorzugt im Bereich von 40-fach bis 65-fach. Verwendet man für die benötigte hohe Auflösung eine Immersionsflüssigkeit, kann die numerische Apertur Werte im Bereich von 1,4 annehmen. Wird die Probe von Luft umgeben, ist die numerische Apertur auf Wert von max. 1, technisch bis 0,95 oder 0,9 begrenzt.

Die bildseitige NA ergibt sich aus der objektseitigen NA über die Vergrößerung NA_Bild = NA_Obj/V.
Die numerische Apertur NA und die f-Zahl (f#) der Optik sind verknüpft über f# = 1/ (2 * NA).
Damit ergeben sich für Vergrößerungen von 40 bzw. 63 und numerischen Aperturen von 0,95 bzw. 1,4, wobei dann entsprechend f# im Bereich von 14 bis 33 liegt.

Bevorzugt handelt es sich beispielsweise bei der Lichtfeldkamera um eine Lichtfeldkamera mit der Bezeichnung Raytrix R12 Micro Series der Raytrix GmbH, Kiel.

Aus dem Lichtfeld lässt sich das Bild des Objektes für unterschiedliche Tiefen rekonstruieren, was unterschiedlichen Fokuseinstellungen des Mikroskops entspricht. Dieses rein digitale Umfokussieren kann an den aufgenommenen Datensätzen im Nachhinein erfolgen.

Der vorteilhafte Analysator hat den Vorteil, dass neben den Farbinformationen RGB auch Tiefeninformationen D (Depth) verfügbar sind, die in einer nachgelagerten computerbasierten Auswertung als weiteres Merkmal analog eines Farbkanals verwendet werden können.

Basierend auf der zusätzlichen Tiefeninformation können die Zellen z.B. einfacher segmentiert, also für die weitere Analyse erkannt und aus dem Bild ausgeschnitten werden. Neben farblichen Kontrastübergängen entstehen dann auch im Höhenprofil an den Rändern der Zellen Übergänge, die gut und genau detektierbar sind.

Bei herkömmlichen Systemen besteht bisher das Problem der sicheren Bestimmung des Randes einer Zelle nur auf dem Farbbild basierend, weil dafür meist ein Schwellwert verwendet wird, der systembeding schon innerhalb der Zelle liegt, da ja zum Erreichen des Schwellwertes schon eine gewisse Veränderung stattgefunden haben muss. Darum werden Zellen meist systematisch zu klein gemessen. Das ist bei Volumenmessungen störend und wird dann z.B. über Interpolationsalgorithmen versucht zu korrigieren. Mit der Bereitstellung und Verwendung der Tiefeninformation können diese Probleme einfach und sicher gelöst werden, da der Hintergrund des Objektträgers eine Ebene darstellt, auf die das Profil abfällt. Es können dann bekannte Methoden zur geometrischen Bestimmung der Zellränder aufgrund von Höhenprofilen entsprechend angewendet werden.

Durch die Aufnahme des Lichtfeldes mit der Lichtfeldkamera kann das aufgenommene Bild, z.B. des Blutausstrichs oder der Zellen, noch offline nach der Bildaufnahme umfokussiert werden. Das kann zum einen genutzt werden, um die Zellen zur Segmentierung und Erkennung für eine Klassifikation bestmöglich zu fokussieren oder auch für die Segmentierung und Klassifikation mehrere Fokusebenen parallel zu verwenden. Alternativ können auch Volumendaten bzw. 3D Punktewolken verwendet werden. Dadurch können die Segmentierung und Klassifikation mit höherer Genauigkeit erfolgen.

Sind die Bilder, z.B. der Zellen, mit der Lichtfeldkamera aufgenommen worden, besteht die Möglichkeit, die Bilder im Nachhinein, also offline, rein digital durch zu fokussieren. Dies würde bei unklaren Klassifizierungen oder Bewertungen von Zellen durch den Computer dem Arzt die Möglichkeit geben, die Zellen unmittelbar im digitalen Bild anzuschauen und nicht erst wieder den Objektträger unter ein Mikroskop zu legen, die Zelle zu suchen und dann über die Fokusvariation eine genauere Bewertung und Klassifizierung vorzunehmen. Da es zwischen dem Zellanalysator und dem Mikroskop keine abgeglichenen Koordinatensysteme gibt, kann der Arzt bisher die zu untersuchende Zelle nur mit einer ungefähren Ortsangabe im Mikroskop positionieren und muss die Zelle dann im Mikroskop final suchen. Das ist zeitaufwendig und muss im Labor erfolgen. Liegt jedoch das digitale Bild der Lichtfeldkamera gemäß der vorliegenden Erfindung vor, kann der Arzt die fragliche Zelle bzw. Struktur unmittelbar im digitalen Bild durchfokussieren.

Diese Bewertung und Klassifizierung ist dann auch im Sinne einer Telemedizin bei einer bestehenden Datenverbindung des Arztes zu dem Bild bzw. der Bilddatenbank möglich, so dass er nicht mehr zum Objektträger im Labor kommen muss.

Bei unklaren Befunden kann so auch eine Konsultation eines Kollegen erfolgen, dem das Bild für die Analyse zugeschickt oder z.B. über elektronische Netzwerke verfügbar gemacht wird, was bei digitaler Datenübertragung quasi in Echtzeit erfolgen kann. Für eine Bewertung und Klassifizierung der aufgenommen Lichtfeldbilder von den Blutzellen benötigt der jeweilige Arzt nur eine entsprechende Analysesoftware auf seinem digitalen Endgerät oder aber im Sinne einer Cloudlösung kann sowohl das Bild der Lichtfeldkamera in der Cloud gespeichert sein als auch die Auswertung z.B. über einen Web-Browser Interface in einer Cloud basierten Anwendung erfolgen.

Bevorzugt bestimmt sich die Vergrößerung aus der Anforderung an die optische Auflösung für die Abtastung des Messobjektes, wobei mit der Wahl der Vergrößerung im Allgemeinen die benötigte effektive Blendenzahl bzw. die numerische Apertur verbunden ist.

Die laterale Auflösung beträgt bevorzugt 100 nm in der Objektebene. Die Vergrößerung ergibt sich dann zusammen mit der lateralen Dimension eines Sensorelements zu V = laterale Dimension Sensorelement / 100 nm.
Diese Vergrößerung ist z.B. besonders vorteilhaft für die Abbildung von Blutzellen.

Etwa 100 nm ist die typische Auflösungsgrenze der Lichtmikroskopie bei Wasser-, Öl oder Glycerinimmersion. Bei einer bekannten Pixelgröße der Kamera von z.B. 4,5 µm wäre somit die benötigte Vergrößerung 45-fach. Die Apertur ergibt sich dann aus der Apertur in der Objektebene - bei Immersion im Bereich größer 1, typisch 1,2 bis 1,4 - geteilt durch die Vergrößerung, also NA Kamera = 1,4 / V z.B. 1,4 / 45 = 0,031. Die Blendenzahl ergibt sich dann aus f# = 1 /2* NA zu f# 16. Bevorzugt beträgt die Blendenzahl der Kamera 2,4, 2,8, 5,6, 7,0 oder 26,0. Besonders bevorzugt beträgt die Blendenzahl der Kamera f# 26 und die Vergrößerung 63-fach.

Bei einer Pixelgröße der Kamera von 2 µm ist eine Vergrößerung von 20x für die angestrebte Auflösung in der Objektebene bevorzugt. Damit wird NA Kamera = 1,4 / 20 = 0,07 und somit die Blendenzahl f# 7. Dies hat den Vorteil, dass mit hochauflösenden Kamerachips mit hoher Pixelzahl große Gesichtsfelder abgedeckt werden können und ferner den Aufwand für die Optik minimiert werden kann.

Bevorzugt umfasst die medizinische Probe eine Zelle und/oder ein medizinisches Präparat. Bevorzugt handelt es sich bei dem medizinischen Präparat um einen Gewebeschnitt, Sedimente aus Körpersekreten und/oder Körperflüssigkeiten und/oder Mikrokristalle.

Besonders bevorzugt handelt es sich bei der Probe um eine Blutprobe und/oder bei der Zelle um eine Blutzelle. Anstelle von Blutzellen kann es sich bei der Probe bevorzugt um jede Art von menschlicher, tierischer oder pflanzlicher Zelle handeln. Dies hat den Vorteil, dass verschiedenste Probentypen, einschließlich verschiedenster Zelltypten, untersucht und charakterisiert werden können.

Die Kombination von einer Lichtfeldkamera mit einem Mikroskop für die Hämatologie wird noch deutlich ergänzt über erweiterte Kontrastierungsmethoden, wie z.B. Phasenkontrast oder Differential-Interferenz-Kontrast (DIC).

Bevorzugt handelt es sich bei dem Mikroskop um ein Amplitudenkontrast-, und/oder ein Phasenkontrast-, und/oder ein Differential-Interferenz-Kontrast (DIC) Mikroskop.

Bevorzugt handelt es sich bei dem Mikroskop um ein differential Digital Holographisches Mikroskop.

Die erweiterten Kontrastierungsmethoden und davon insbesondere der DIC erlaubt es, Phasenunterschiede der Lichtwege durch die Probe, also hier z.B. die Zellen, sichtbar zu machen. Unterschiedliche Phasenwerte können insbesondere beim Phasenkontrast dann z.B. mit unterschiedlichen Farben dargestellt werden und so mit einer Farbkamera gemessen werden. Dadurch ist es möglich, auf ein Färben der Proben zu verzichten und dennoch die Zellen gut kontrastiert abzubilden. Bisherige Systeme zur automatisierten Zellklassifizierung nutzen ausschließlich den Amplitudenkontrast, der durch die Färbung der Zellen und die unterschiedlichen Laufwege des Lichts in der Zelle bzw. alternativ dem umgebenden Medium entsteht.

Durch die Kombination eines Mikroskops und einer Lichtfeldkamera erhält man zusätzliche 3D Informationen zur Zelle, die für eine Klassifizierung der Zelle herangezogen werden können. Je nach gewählter Kontrastierungsmethode im Mikroskop werden somit folgende Daten für eine Klassifizierung der Zellen erhalten. Die Klassifizierung kann z.B. von medizinischem Fachpersonal und/oder computerbasierten Systemen durchgeführt werden. Im Falle von Amplitudenkontrast liegen herkömmliche Bildinformation sowie zusätzlich 3D Information der Zelle vor. Bei Phasenkontrast wird ein Phasenbild sowie zusätzlich 3D Information der Zelle erhalten. Bei DIC wird ein DIC-Bild zu differentiellen Phasenbildern sowie zusätzlich 3D Information der Zelle erhalten.

Erfindungsgemäß kann die erhaltene 3D Information der Zelle vorteilhafterweise z.B. unterschiedlich wie folgt repräsentiert werden.

Vorteilhafterweise wird die 3D Information der Zelle als RGB Bild dargestellt. Vorteilhafterweise ist RGB Bild als sogenanntes Total Fokus Image über den gesamten Tiefenschärfebereich der Lichtfeldkamera scharf abgebildet. Durch den im Vergleich zu herkömmlichen 2D Kameras vergrößerten Tiefenschärfebereich wird damit mehr Tiefeninformation erfasst. Eine Blutzelle hat - je nach Art und Ausrichtung - eine Dicke von etwa 1 bis 2 µm, bis ca. 20 µm. Die Schärfentiefe der Abbildung ist bei NA 1,3 und bei 500 nm Wellenlänge allerding nur d = ± λ/NA^ 2 = ± 500 nm/1,69 = ± 300 nm. Somit ist im herkömmlichen 2D Bild keine der Zellen über die volle Tiefe scharf abgebildet. Die Schärfentiefe vergrößert sich um mindestens den Faktor vier über den plenoptischen Effekt aus der Lichtfeldkamera. Somit werden Zellen, insbesondere z.B. rote Blutzellen (RGB) vollständig scharf abgebildet. Auch werden größere Zellen, wie z.B. weiße Blutzellen (WBC), zu einem wesentlich größeren Teil des Zellvolumens scharf abgebildet.

In einer weiteren vorteilhaften Ausgestaltung wird die 3D Information der Zelle als RGB D Information dargestellt. Jeder Bildpunkt enthält eine als D bezeichnete Tiefeninformation. Wenn als Probe z.B. Blutzellen beispielsweise auf einem Objektträger aufgebracht sind, ist D z.B. die Dicke der Blutzelle. Diese Information ergänzt die Farbinformation und wird auch als 3D Punktewolke bezeichnet.

In einer weiteren vorteilhaften Ausgestaltung wird die 3D Information der Zelle als volumetrische 3D Information dargestellt. Analog einem Bild eines Computer-Tomograph (CT) entstehen so räumliche Bildinformationen in Form von Voxeln. Da die Zellen, z.B. Blutzellen, für die Strahlung zumindest teilweise transparent sind, können so unterschiedliche Stellen der Zelle Streustrahlung generieren, die von der Kamera aufgenommen und unterschiedlichen Tiefen zugeordnet wird.

In einer weiteren vorteilhaften Ausgestaltung kann aus dem Datensatz der Lichtfeldkamera ein Bildstapel berechnet werden, der auf unterschiedliche Fokusebenen berechnet ist und so die volumetrische Information enthält. In der Auswertealgorithmik der Lichtfeldkamera bieten sich für die Ebenen, die sogenannten virtuellen Depth, als Abstandsmaß an. Alternativ können die Fokusebenen auch mit anderen Abstandwerten berechnet werden, die beispielsweise äquidistant gewählt sind und von denen eine Ebene mit dem max. Querschnitt der Zelle zusammen fällt.

Im Falle der Kontrastierung mit Phasenkontrast basiert das Bild der Lichtfeldkamera nur auf den von der Phasenwirkung erzeugten Intensitätsmodulationen, da die Amplituden- bzw. Intensitätsmodulation aus dem Objekt selber im Phasenring ausgefiltert werden. Dieses Bild ist weitestgehend farbecht zum Bild mit reinem Amplitudenkontrast.

Im Falle der Kontrastierung mit DIC basiert das Bild der Lichtfeldkamera auf einem Bild der Zellen, das über den differentiellen Interferenzkontrast die Information zu den unterschiedlichen Laufwegen des Lichts beim Weg durch die Zelle und damit der Phasenänderung des Lichts als farbkodierte Information enthält. Dies bietet den Vorteil, dass die Farbdarstellung der Zelle eine feine Struktur überlagert, die für die Auswertealgorithmen der Lichtfeldkamera sehr hilfreich sind für eine hohe laterale Auflösung bei der Berechnung der Tiefeninformation.

In einer vorteilhaften Ausgestaltung werden bis zu vier Farbkanälen mit RGB und weiß verwendet.

Vorteilhafterweise umfasst das Spektrum der Beleuchtung den sichtbaren Bereich und/oder nahe IR Wellenlängen.

Bevorzugt erfolgt ein Farbabgleich der Lichtquelle zu einer Kamera, z.B. der Lichtfeldkamera. Weiter erfolgt bevorzugt eine Anpassung der Belichtungszeit und des gain für die 3-Chip RGB Farbkamera für die 2D Bilder. Besonders bevorzugt umfasst die Lichtquelle eine 4 LED-Lichtquelle mit RGB und W sowie einer gemeinsamen Helligkeitsregelung.

In einer weiteren bevorzugten Ausführungsform der Erfindung ist bei dem Analyzer an dem Mikroskop eine weitere Kamera zum Aufnehmen eines Bildes in einer Objektebene im Objektbereich vorgesehen, wobei die weitere Kamera eine laterale Auflösung aufweist, die gleich oder bevorzugt höher als die laterale Auflösung der Lichtfeldkamera ist, wobei die Auflösung der weiteren Kamera die doppelte, bevorzugt die dreifache, besonders bevorzugt die vierfache Auflösung der Lichtfeldkamera beträgt.

Bevorzugt weist die weitere Kamera ein größeres Gesichtsfeld, auch als Field of view bezeichnet, als die Lichtfeldkamera auf. Dies ermöglicht eine bessere und schnellere Probenabdeckung.

Bevorzugt ist die Fokusebene der weiteren Kamera, bevorzugt einer 2D Kamera, mit einer ausgezeichneten Ebene der Lichtfeldkamera gekoppelt. Dies kann beispielsweise in virtual depth gemessen werden. So kann vorteilhafterweis erreicht werden, dass beide Kameras gleichzeitig gute Bilder liefern.

In einer weiteren bevorzugten Ausführungsform der Erfindung ist die weitere Kamera eine Farbkamera, bevorzugt eine Mehrchip-Farbkamera, besonders bevorzugt eine 3-Chip Farbkamera.

Dies hat den Vorteil, dass mittels der hochauflösenden Farbkamera, z.B. einer hochauflösenden RBG Kamera, eine hohe Auflösung gewährleistet ist. Dies ist von besonderem Vorteil, da bei der Lichtfeldkamera Prinzip bedingt lateral ein Faktor zwei, flächig ein Faktor vier an Auflösungsvermögen verloren geht, um Informationen zur Berechnung der Tiefenauflösung zu gewinnen. Dieser Verlust an Auflösungsvermögen kann so vorteilhafterweise vollständig kompensiert werden.

Die Verwendung einer Mehrchip-Farbkamera, z.B. einer 3-Chip Farbkamera, hat den Vorteil, dass dann auch eine bessere Farbmessung und insbesondere pixelweise Farbbestimmung ohne Interpolation mit mehr Dynamik möglich ist, was weitere Vorteile für die Klassifizierung von Zellen bringt. Vorteilhafterweise erfolgt ein Abgleich von Farbkanälen für ein optimiertes S/N-Verhältnis. Bei z.B. gefärbten Proben erfolgt der Abglich bevorzugt für jedes Färbeprotokoll getrennt. Vorteilhafterweises ist die 3-Chip Farbkamera eine 3-Chip CMOS-Kamera mit 3,2 Mega-Pixeln pro Chip.

Eine 3-Chip Farbkamera wird gegenüber z.B. einer 1-Chip Farbkamera bevorzugt, da eine 1-Chip Farbkamera meist ein Bayer-Pattern einsetzen. Da es bei der Analyse von z.B. Blutzellen in einem Hämatologieanalysator z.B. besonders auf gute Farbauflösung und Farbechtheit und auch eine gute Strukturauflösung lateral ankommt, sind hier die 3-Chip Kamera bevorzugt. Die hochauflösende Farbkamera liefert z.B. ein RGB Bild mit hoher Auflösung mit typischerweise besseren, d.h. genaueren Farbwerten und geringerem Rauschen, wenn man die Belichtung für jeden der Farbkanäle getrennt optimiert. Im Falle der 3-Chip Farbkamera werden die Farbwerte unmittelbar und ohne Interpolation für jedes Pixel getrennt bestimmt.

Bevorzugt werden anschließend noch die Farbwerte einer Farbkamera von der RBG-Darstellung auf z.B. eine HSV-Darstellung für Farbwert (Hue), Sättigung (Saturation)und Helligkeit (Value) übertragen, so lassen sich dann beispielsweise rote Blutkörperchen, wo der rote Farbstoff homogen in der Zelle verteilt ist, auch gut über den Farbwert als weitere oder ergänzende Methode segmentieren. Dieser Ansatz funktioniert für alle Zellen und Struktur gut, die homogen den gleichen Farbwert (Hue) haben bzw. mit der gleichen Farbe gefärbt sind. Diese Segmentierung kann vorteilhafterweise mit der über die Tiefenwerte (D) kombiniert werden, um so ein möglichst präzises Kriterium zu haben, die Zelle auszuschneiden.

Die Bilder der beiden Kameras werden vorteilhafterweise über Skalierung und/oder Pixelinterpolation zueinander registriert, falls die effektiven Pixelgrößen in den Bildern nicht entsprechend zusammen passen oder kommensurabel sind mit ganzzahligem Faktor von z.B. 1, 2, 3, etc. Hierfür sind laterale Verschiebungen, Verdrehungen, Verkippungen, Verzeichnungen und/oder Verzerrungen in den Bildern und/oder auch ein Defokus prinzipiell vorteilhafterweise zu korrigieren. Wenn die Bilder dann in dem erforderlichen Umfang registriert sind, werden neue Bilddaten berechnet, die die höhere laterale Farbauflösung mit der Tiefeninformation aus dem Bild der Lichtfeldkamera kombinieren. Dazu kann das Bild der Farbkamera an die zugeordnete Fokusposition im ausgewerteten Tiefenbild der Lichtfeldkamera gesetzt werden und von dort aus über die aus der Aufnahme der Lichtfeldkamera bekannte Propagation des Lichtfeldes auch die Farbdarstellungen und lateralen Auflösungen auf benachbarte Fokusebenen übertragen werden.

Die Übertragung kann dabei beispielsweise lateral über Interpolation geschehen, für die Farben z.B. über Korrespondenztabellen. Alternativ ist eine aufwendigere Übertragung auch über eine echte Propagationsrechnung basierend auf dem von der Lichtfeldkamera gemessenen Daten zusammen mit z.B. einem Phase-Retrieval möglich. Dabei dienen ausgewertete Bildebenen der Lichtfeldkamera als Stützstellen und die weiteren zusätzlichen Punkte werden über die Nachbarpunkte abgestützt.

Basierend auf der einen ausgezeichneten Ebene und zusammen mit geeigneten Stetigkeitsbedingungen für die optischen Felder und die zugehörigen berechneten optischen Wellenfronten ist eine vergleichsweise genaue und umfangreiche Interpolation möglich.

Vorteilhafterweise wird diese genauere Lösung, die typischerweise sehr rechenaufwendig ist, für eine Feindiagnostik, z.B. an auffälligen Bildbereichen, Zellen mit unklarem Befund und/oder pathologischen Zellen eingesetzt, wo es weniger oder nicht auf Echtzeitfähigkeit ankommt. Die Feindiagnostik kann dann auch beispielsweise am kompletten Bild oder auch nur an Bildausschnitten erfolgen, z.B. an einzelnen segmentierten Zellen gegebenenfalls erweitert um gewisse Randbereiche darum.

Je nach Anordnung im Strahlengang sind folgende unterschiedliche, jeweils vorteilhafte Varianten zur Anordnung erfindungsgemäß vorgesehen.

Für die Vermessung von gefärbten Zellen oder Präparaten oder solchen, die in den Bildern einen guten Kontrast ohne erweiterte Kontrastierungsmethoden liefern, können die beiden Kameras gleichzeitig am Mikroskop verwendet und betrieben werden. Bei lebenden oder bewegten Zellen oder Proben oder Untersuchungen unter Nutzung von mikrofluidischen Zellen kann es vorteilhaft sein, wenn die beiden Kameras auch zeitlich synchron die Bilder aufnehmen.

Hierbei können z.B. handelsübliche Mikroskoptuben verwendet werden, die mechanisch den parallelen Betrieb von zwei Kameras an einem Mikroskop ermöglichen. Es gibt hierbei verschiedene Arten der Strahlteilung, z.B. das Aufspaltungsverhältnis, die Aufspaltungsmethode wie spektrale Trennung, Polarisationstrennung etc., die entsprechend vorteilhaft gewählt werden können.

Bei ungefärbten Zellen, die erweiterten optischen Kontrastierungsmethoden bedürfen, sind bei dem erfindungsgemäßen Verfahren weitere bevorzugte technische Ausführungen des Analyzers erfindungsgemäß optional vorgesehen.

Bevorzugt wird nur eine Polarisationsrichtung für das Farbbild genutzt. Hierzu wird eine Trennung der Strahlengänge für die Farbkamera und die Lichtfeldkamera zwischen dem Objektiv und dem DIC-Analysator oder alternativ zwischen dem Objektiv und vor dem objektivseitigen DIC-Prisma vorgesehen.

Bevorzugt wird die Teilung des Lichtfeldes auf der Abbildungsseite bereits bei der Ausrichtung der Polarisation vor der Aufspaltung im Beleuchtungsmodul so berücksichtig, dass nach dem DIC Prisma und DIC Analysator die beiden Polarisationsanteile das gewünschte Intensitätsverhältnis, wie z.B. 1:1 für maximalen Kontrast, haben. Auf diese Weise können Lichtverluste minimiert werden, um so die Effizienz des Gesamtaufbaus zu optimieren und die benötigten Belichtungszeiten für eine schnelle Messung zu minimieren.

Bevorzugt erfolgt die Trennung des Strahlengangs auf die beiden Kameras zwischen dem Abbildungsobjektiv und dem DIC-Analysator mit einem polarisationsneutralen Strahlteiler. Im Strahlengang zur hochauflösenden Farbkamera ist dann noch ein Polarisator so anzuordnen, dass er die eine Polarisationsrichtung des DIC Strahlenganges möglichst vollständig sperrt und die andere mit möglichst geringer Abschwächung auf die Kamera passieren lässt. Dieser Polarisator kann entweder in Transmission oder in Reflektion arbeiten oder zu einer räumlichen Trennung der unterschiedlich polarisierten Strahlen führen. Dies bietet den Vorteil, dass der Aufbau weniger Komplex in der Auslegung ist und keine besondere polarisationsabhängige Justage benötigt, jedoch gehen Prinzip bedingt ca. 25 % der Lichtmenge verloren.

Vorteilhafterweise sind die Bilder der Lichtfeldkamera und der Farbkamera zueinander passend ausgerichtet und skaliert, damit z.B. in einem Topografiebild die 3D- bzw. Höheninformation der Lichtfeldkamera genutzt werden kann und zugleich die Farbinformation von der Farbkamera herangezogen und genutzt werden kann.

Bevorzugt erfolgt die Aufnahme mittels der Lichtfeldkamera und der Farbkamera zeitgleich. Dies kann z.B. durch eine entsprechende Triggerung erreicht werden. Die Triggerung kann über eine Hardware oder auch über eine Software ausgeführt sein. Die Triggerung kann auch von einer ausgewählten ersten Kamera auf die zweite Kamera erfolgen, was auch als Master-Slave-Kopplung bezeichnet wird. Funktional muss sichergestellt sein, dass die Bilder mit einem vorgegebenen zeitlichen Bezug, idealerweise zeitgleich, aufgenommen werden. Durch mögliche Latenzzeiten in der Hard- und Software der Kameras und der jeweiligen Ansteuerung können die Triggersignale selber einen zeitlichen Abstand aufweisen. Die zeitgleiche Aufnahme ist von besonderem Vorteil für die Aufnahme von bewegten Zellen, z.B. in einer Durchflusszelle, und/oder lebenden Zellen bzw. Messobjekten.

Vorteilhafterweise erfolgt eine Kombination der Bilder von der Lichtfeldkamera und der hochauflösenden, weiteren Kamera nur für Bildbereiche ausschnittsweise, z.B. nur in einem Bereich, in dem in einem automatisierten Messprozess zu untersuchende Messobjekte erkannt oder detektiert wurden. Dies hat den Vorteil, dass eine erhöhte Messgeschwindigkeit erreicht werden kann, da entsprechend Rechenaufwand und Rechenzeit eingespart werden kann.

Je nach Anordnung im Strahlengang können unterschiedliche Ausgestaltungen vorteilhaft sein.

In einer vorteilhaften Ausgestaltung wird nur eine Polarisationsrichtung für das Farbbild genutzt und die Trennung der Strahlengänge für Farbkamera und Lichtfeldkamera erfolgt noch vor dem DIC-Analysator oder bereits vor einem objektivseitigen DIC-Prisma. Die Teilung des Lichtfeldes kann bei der Ausrichtung der Polarisation vor der Aufspaltung im Beleuchtungsmodul vorteilhafterweise so berücksichtig werden, dass nach dem DIC Prisma und DIC Analysator die beiden Polarisationsanteile das gewünschte Intensitätsverhältnis, wie z.B. 1:1 für maximalen Kontrast, haben. Dies ist insbesondere dann besonders vorteilhaft, wenn angefärbte Zellen abgebildet und analysiert werden sollen.

In einer weiteren vorteilhaften Ausgestaltung wird die Farbkamera für hochaufgelöstes DIC und die Plenoptik für einen erweiterten Tiefenmessbereich für eindeutiges unwrapping der Farbbereiche zur Messung von dickeren Zellen oder Zellclustern genutzt. Eine hochaufgelöste relative Dickenmessung erfolgt also mittels DIC indem die differentiellen Kontrastinformationen z.B. aufintegriert werden und eine großräumigere Zuordnung erfolgt mittels Plenoptik.

Bei ungefärbten Proben, z.B. Zellen, enthält der Amplitudenkontrast kaum bis keine Information und das Farbbild wird vorteilhafterweise nur für Phasen- bzw. DIC-Kontrast herangezogen, der dann jedoch eine hohe laterale Auflösung aufweist mit einer echten Farbtrennung pro Pixel.

In einer vorteilhaften Ausgestaltung sind die Lichtfeldkamera und die hochauflösende Kamera, die vorteilhafterweise eine Farbkamera umfasst, relativ zueinander ausgerichtet bezüglich der Achsrichtungen des Sensorarrays und vorteilhafterweise auch dem subpixel genauen shift entlang der Achsenrichtungen des Arrays und/oder einem richtungsabhängigen Skalierungsfaktor für z.B. die x- und/oder y-Achse.

In einer vorteilhaften Ausgestaltung ist die Lage des Fokus der hochauflösenden Kamera auf den mittleren Messbereich der Lichtfeldkamera eingestellt. Dies hat den Vorteil, dass die Lichtfeldkamera prozessual auch ähnlich eines Autofokussystems genutzt werden kann.

Bei kleinerer effektiver Blendenzahl f# erhöht sich die erreichbare Tiefenauflösung der Lichtfeldkamera. So kann eine Blendenzahl von 7 deutlich vorteilhafter sein als eine Blendenzahl 26. Eine geringere Blendenzahl bedeutet eine höhere bildseitige Apertur. Da die objektseitige Apertur bei den für biologische Proben typischen Immersion auf 1,4 beschränkt ist, ergibt sich die maximale bildseitiger Apertur zu NA Kamera = 1,4/V.

Vorteilhafterweise ist am Mikroskop schon das Objekt mit der vollen Abbildungsapertur beleuchtet, um bildseitig die volle Apertur der working f# auszuleuchten damit die Lichtfeldkamera gut funktioniert und eine hohe laterale Abtastung bereitstellen kann. Günstigenfalls ist NA Beleuchtung = NA Objektiv. Das wird auch als inkohärente Beleuchtung mit Sigma = o = NA Beleuchtung / NA Objektiv = 1 bezeichnet.
In einer vorteilhaften Ausgestaltung des erfindungsgemäßen automatischen Analyzers ist am Mikroskop die inkohärente Beleuchtung Sigma größer als 0,8, bevorzugt größer als 0,9, besonders bevorzugt 1,0, wobei Sigma gegeben ist als der Quotient aus der numerischen Apertur der Beleuchtung der Probe und der numerischen Apertur des Objektives und wobei es sich bei dem Mikroskop bevorzugt um ein Differential-Interferenz-Kontrast (DIC) Mikroskop handelt.

In der Hämatologie ist dieses σ = 1 bzw. σ > 0,8 oder besser o > 0,9 sehr wichtig, da es sich bei den Blutzellen um schwach streuende Objekte handelt und somit die Apertur auch für ein Mikrolinsenarray in der Lichtfeldkamera ausreichend mit Licht gefüllt wird.

Bisher wurden in der Regel in der Lichtmikroskopie zur Optimierung des Kontrastes - insbesondere für die visuelle Beobachtung, aber auch bei Kameras - Beleuchtungen nach Köhler eingesetzt mit einem Sigma = 0,7. Dies würde jedoch hier zu einer nicht hinreichend mit Licht gefüllt Apertur führen und somit könnte keine genauere Tiefenbestimmung erfolgen. Zudem würde wegen der nicht ausgeleuchteten Pixel ein Großteil der räumlichen Auflösung verloren gehen. Dies hängt damit zusammen, dass die verwendete Pixelzahl für die Berechnung der Bilder näherungsweise quadratisch mit dem Wert
von Sigma ansteigt und bei σ = 1 das Maximum nahe der vollständigen Detektorauflösung erreicht.

In einer vorteilhaften erfindungsgemäßen Ausgestaltung des Analyzers umfasst der Analyzer eine Probenzuführungseinrichtung für Objektträger, mittels der dem Analyzer Proben auf einem Objektträger zugeführt werden können.

In einer vorteilhaften erfindungsgemäßen Ausgestaltung des Analyzers umfasst der Analyzer eine Durchflusszelle für die Zuführung der Probe, wobei bevorzugt die Objektebene des Mikroskops in der Durchflusszelle liegt. Bevorzugt handelt es sich bei der Durchflusszelle um eine mikrofluidische Durchflusszelle.

Dies hat den Vorteil, dass insbesondere Zellen oder Blutzellen in der natürlichen Umgebung abgebildet und analysiert werden können. Es kann insbesondre vermieden werden, dass die Zellen mittels Ausstrich auf einem Objektträger aufgebracht werden müssen, wo sie häufig getrocknet und/oder gefärbt werden, was ihre ursprünglichen natürlichen Eigenschaften, wie z.B. Form deutlich verändern kann. Weiter entfällt der entsprechende Präparationsaufwand für das Herstellen von Ausstrichen und das Anfärben und es wird im erheblichen Maße Abfall vermieden durch den Wegfall des Verbrauchs von z.B. Objektträgern und Deckgläschen für die Ausstriche. Ebenfalls werden Aufbewahrungskapazitäten bezüglich der Ausstriche und der Verbrauchsmaterialien vermieden. Auf Systemebene des Analyzers können die Vorrichtungen zum automatischen Wechseln der Ausstriche entfallen, was u.a. eine erhebliche Vereinfachung des Geräteaufbaus ermöglicht.

Da die nutzbare Schicht in einer Durchflusszelle typischerweise mehrere Mikrometer dick ist, teilweise auch einige 10 µm dick, ist es schwierig die Zellen genau in der Objektebene der für die Untersuchung genutzten Mikroskop Optik zu positionieren. Diesbezüglich ergibt sich eine gegenläufige Anforderung von dem Wunsch nach hoher Auflösung einerseits und der nicht so präzisen Lage der Zelle in Richtung der optischen Achse andererseits.

Bevorzugt kann hier die Tiefenmessung der Lichtfeldkamera dann zum Optimieren der Ansteuerparameter der Mikrofluidik dienen, um so z.B. den Fokus passend einzustellen, dass auch die 2D hochauflösenden Bilder scharf sind. Eine Belichtung und 3D Bilder ist auch ein Mehrwert ohne Interferometrie, wie bei DHM, was aufwendig und komplex ist und z.T. Störungen durch Effekte mit zeitlich und/oder räumlich kohärenter Strahlung hat.

Mit steigender numerischer Apertur NA der Optik wird die laterale Auflösung besser über die Beziehung δ = 0,5 λ/NA. Andererseits verkleinert sich mit der numerischen Apertur die Schärfentiefe gemäß d = ± λ/NA^2. Bei 500 nm Wellenlänge und NA = 0,7 ergibt sich dann eine Schärfentiefe von insgesamt 2 µm, was z.B. ungefähr der Dicke roter Blutkörperchen entspricht.

Bei den bisher gebräuchlichen Geräten wird mit einer Optik mit Aperturen von NA = 0,5 gearbeitet, was dann bei 500 nm einer Schärfentiefe von insgesamt d=4 µm entspricht. Über die NA = 0,5 ist die erreichbare laterale Auflösung auf δ = 0,5 µm begrenzt. Bisher musste also ein entsprechender Kompromiss mit der Wahl der numerischen Apertur zum Ausgleich zwischen lateraler Auflösung und Schärfentiefe gewählt werden. Die genannten Beziehungen beschreiben in der gängigen Theorie das Auflösungsvermögen optischer Instrumente.

In einem erfindungsgemäßen automatischen Analyzer mit einer entsprechenden Lichtfeldkamera kann der Tiefenmessbereich, also der Bereich, wo scharfe Bilder aufgenommen werden um einen Faktor von etwa 4 bis etwa 6 vergrößert werden. Im Gegenzug sinkt die laterale Auflösung der Lichtfeldkamera um einen Faktor 2.

Da bei der Auswahl der verwendeten Optik die Vergrößerung und die Apertur weitgehend unabhängig voneinander gewählt werden können, kann dieser Effekt jedoch gut kompensiert werden. Wählt man z.B. eine größere Vergrößerung und auch eine höhere Apertur, so lässt sich der Effekt entsprechend positiv verwenden.

Wird beispielsweise eine Vergrößerung von 60-fach bis 80-fach und eine Apertur von NA 0,7 gewählt, ergeben sich überschlagsmäßig für 500 nm Wellenlänge die folgenden Werte. NA = 0,7, δ = 0,36 µm, Schärfentiefe d = 1,05 µm. Mit dem Effekt zur Vergrößerung des Tiefenarbeitsbereichs aus der plenoptischen Kamera ergeben sich Werte für eine erhöhte Schärfentiefe von d = 5,25 µm, unter Annahme einer Erhöhung um etwa einen Faktor 5.

Wird beispielsweise eine Vergrößerung von 60-fach bis 80-fach und eine Apertur von NA 0,9 gewählt, ergeben sich überschlagsmäßig für 500 nm Wellenlänge die folgenden Werte. NA = 0,9, δ = 0,28 µm, Schärfentiefe d = 0,62 µm. Mit dem Effekt zur Vergrößerung des Tiefenarbeitsbereichs aus der plenoptischen Kamera ergeben sich Werte für eine erhöhte Schärfentiefe von d = 3,10 µm, unter Annahme einer Erhöhung um etwa einen Faktor 5.

Mittels des Einsatzes der Lichtfeldkamera in einem Zellanalysator kann also der Tiefenarbeitsbereich stark vergrößert werden, was dann entsprechend die Verwendung einer Durchflusszelle ermöglicht. Es wird insbesondere eine vollständigere oder vollständige Erfassung der Ausdehnung einer Zelle über die Höhenausdehnung der Strömung der Durchflusszelle möglich. Dies ermöglicht insbesondere präzise Untersuchungen an typischerweise ungefärbten Zellen. Für bestimmte Untersuchungen kann es jedoch auch vorteilhaft oder notwendig sein, dass die Zellen in dem Medium der Mikrofluidik angefärbt werden.

Ein weiterer Vorteil ist, dass die Parameter der Durchflusszelle in Bezug auf Fokussierung und/oder Lage der Zelle in einem weiteren Parameterbereich liegen können und z.B. auf eine entsprechende spezielle Optimierung verzichtet werden kann.

In bevorzugter Ausgestaltung umfasst das Gesichtsfeld des Mikroskops die volle Breite der Durchflusszelle, durch welche Zellen strömen können. Dieser Bereich hat vorteilhafterweise eine Breite von wenigen 1/10 mm bis zu wenigen mm.

In einer bevorzugten Ausführungsform des automatischen Analyzers umfasst der Analyzer sowohl Mittel, um Objektträger mit Zellen anschauen zu können und/oder um Zellen in der Durchflusszelle anschauen zu können. Vorteilhafterweise sind dabei die Abmessungen der Durchflusszelle auf die Maße der Objektträger, insbesondere deren Dicke und damit der optischen Wirkungen, abgestimmt. Die Abdeckung der Durchflusszelle- also die Deckscheibe und ggf. auch fluidische Medien, die sich zwischen dem Bereich bzw. der Ebene der Zellen und der äußeren Oberfläche der Durchflusszelle befinden, haben vorteilhafterweise ebenfalls optisch die gleiche Wirkung, z.B. bezüglich optischer Weglängen, Dispersion und/oder Brechungsindex, so dass dieselben Optiken verwendet werde können und die gleiche bestmögliche Abbildungsqualität erhalten bleibt. Bevorzugt kann ein Umfokussieren erfolgen, um sich z.B. an Fertigungstoleranzen anzupassen. Typische Deckglasdicken sind im Bereich unter 0,2 mm, typisch 0,15 bis 0,17 mm. Die lateralen Abmessungen von Objektträgern sind beispielsweise 76 mm mal 26 mm oder 75 mm mal 25 mm gemäß DIN ISO 8037-1 bei einer
Dicke im Bereich von 1 mm bis 1,5 mm. Die Abmessungen der Durchflusszelle ergeben sich dann vorteilhafterweise entsprechend.

In bevorzugter Ausgestaltung ist die Bewegungsgeschwindigkeit der Zellen in der Durchflusszelle im Abbildungsbereich des Mikroskops an die Belichtungszeit der eingesetzten Bildaufnahmesysteme angepasst. Die Bewegung ist dabei typischerweise kleiner als ½ Pixel (pxl), bevorzugt kleiner als 1/5 pxl, besonders bevorzugt kleiner als 1/10 pxl. Dies hat den Vorteil, dass Motion-Blurring-Effekte verringert oder ganz vermieden werden können.

In einer weiteren vorteilhaften Ausgestaltung des Analyzers umfasst der Analyzer eine Probenzuführungseinrichtung für Objektträger. Dies kann besonders dann vorteilhaft sein, wenn z.B. Gewebeschnitte oder andere medizinische Präparate untersucht werden sollen.

In einer vorteilhaften Ausgestaltung umfasst das erfindungsgemäße Verfahren zur virtuellen Annotation einer Zelle, von Bestandteilen einer Zelle und/oder eines biologischen Präparats und/oder das Verfahren zur automatisierten Erkennung, Identifikation und/oder Klassifizierung einer Zelle, von Bestandteilen einer Zelle und/oder eines biologischen Präparats weiter folgende Schritte eines Verfahrens zum Ermitteln einer zweidimensionalen und/oder dreidimensionalen Information einer Zelle mittels eines Analyzers:
a1) Zuführen einer Probe mit einer Zelle zu dem Mikroskop,
b1) Aufzeichnen der von der Zelle in der beleuchteten Probe ausgehenden Lichtstrahlen mittels des digitalen Aufzeichnungsgeräts,
c1) Abbildung des im Mikroskop abgebildeten Lichtfelds aus dem Objektbereich mittels der Lichtfeldkamera, wobei die Lichtfeldkamera das digitale Aufzeichnungsgerät umfasst.
d1) Ermittlung einer zweidimensionalen und/oder dreidimensionalen Information der Zelle aus in Schritt b1) aufgezeichneten Informationen der Lichtstrahlen und/oder Informationen des in Schritt c1) abgebildeten Lichtfeldes.

Vorteilhafterweis können Schritte b1) und c1) auch zu einem Schritt verbunden werden, wobei der Schritt dann umfasst
- Abbildung des im Mikroskop abgebildeten Lichtfelds aus dem Objektbereich mittels der Lichtfeldkamera und
- Aufzeichnen der von der Zelle in der beleuchteten Probe ausgehenden Lichtstrahlen mittels des digitalen Aufzeichnungsgeräts, wobei die Lichtfeldkamera das digitale Aufzeichnungsgerät umfasst

Bevorzugt werden in Schritt b1) die von der Zelle in der beleuchteten Probe ausgehenden Lichtstrahlen zunächst fokussiert und dann mittels des digitalen Aufzeichnungsgeräts aufgezeichnet. Bevorzugt erfolgt eine Konversion der Photonen in elektrische Ladung mit anschließender Bestimmung einer Ladungsmenge und Digitalisierung des Wertes für die Ladungsmenge.

Anstelle von der Zelle kann bevorzugt auch von einem medizinischen Präparat eine zweidimensionale und/oder dreidimensionale Information entsprechend ermittelt werden.

Bevorzugt wird die Probe in Schritt a1) mittels einer Durchflusszelle dem Analyzer zugeführt, wobei die Objektebene des Mikroskops in der Durchflusszelle liegt. Bevorzugt umfasst die Durchflusszelle Mittel, die es ermöglichen, durch eine entsprechende Ansteuerung der Durchflusszelle die Zellen in einer ausgezeichneten Ebene in der Durchflusszelle anzureichern. Bevorzugt ist die Objektebene auf die ausgezeichnete Ebene eingestellt, so dass die Objektebene und die ausgezeichnete Ebene vorteilhafterweise zusammen fallen.

Bevorzugt wird die Probe in Schritt a1) mittels einer Probenzuführungseinrichtung für Objektträger dem Analyzer auf einem Objektträger zugeführt.

Bevorzugt umfasst das Verfahren weiter einen Schritt, in dem ein Bild in einer Objektebene im Objektbereich mittels einer weiteren Kamera zum Aufnehmen eines Bildes in der Objektebene im Objektbereich vorgesehen ist, wobei die weitere Kamera eine laterale Auflösung aufweist, die höher als die laterale Auflösung der Lichtfeldkamera ist, wobei die Auflösung der weiteren Kamera bevorzugt die doppelte, besonders bevorzugt die vierfache Auflösung der Lichtfeldkamera beträgt und wobei bevorzugt die weitere Kamera ein größeres Gesichtsfeld als die Lichtfeldkamera aufweist.

Bevorzugt ist die weitere Kamera eine Farbkamera, bevorzugt eine 3-Chip Farbkamera.

Bevorzugt sind die Zellen und/oder die medizinischen Präparate nicht angefärbt. In diesem Fall sind die erweiterten Kontrastierungsmethoden, wie z.B. Phasenkontrast und/oder differenzieller Interferenzkontrast, insbesondere zur Abbildung von Zellen, besonders vorteilhaft. Bei z.B. Sedimenten oder anderen Proben kann gegebenenfalls auch Amplitudenkontrast vorteilhaft sein.

In einer bevorzugten Ausgestaltung des Verfahrens umfasst das Verfahren zum Ermitteln einer zweidimensionalen und/oder dreidimensionalen Information einer Zelle mittels eines Analyzers weiter den Schritt:
e1) digitale Refokussierung oder Fokusvariation mittels der ermittelten zweidimensionalen und/oder dreidimensionalen Information der Zelle entlang der optischen Achse des Mikroskops, wobei bevorzugt die digitale Refokussierung rechnergestützt und/oder numerisch erfolgt.

Dies hat den Vorteil, dass durch die erfindungsgemäß vorgesehene Refokussierung oder Fokusvariation erstmals eine telemetrische Befundung in der Hämatologie ermöglicht wird. Somit werden auch konsiliarische Befundungen z.B. durch Experten an anderen Standorten oder auch eine Nachbefundung ermöglicht. Um dies mit der etablierten hohen Qualität der Befundung gemäß des Standard of care durchzuführen, ist es unerlässlich, dass dem Nachbefundenden oder konsiliarischen Arzt eine Möglichkeit zur Fokussierung durch die Zellebene hindurch zur Verfügung gestellt wird.

Im Stand der Technik wurde bisher der entsprechende Objektträger mit den betreffenden fraglichen Zellen körperlich aus z.B. einem Magazin, einer Lagerdose oder einem Archiv herausgeholt und dann unter ein Mikroskop gelegt und das Bild jeweils auf die jeweilige Zelle fokussiert. Anhand relativ ungenauer Ortsinformationen bezüglich der Position der jeweiligen Zellen auf dem Objektträger wurde dann versucht, die jeweiligen Zellen wieder aufzufinden. Falls die Zellen wieder gefunden wurden, wird die Befundung vorgenommen. Für die Befundung selbst analysiert der Arzt das optische Bild der Zellen und fokussiert meist auch durch die Zelle hindurch. Daher sind die bisher bekannten Systeme und Methoden für die detaillierte Nachbefundung nicht oder nur sehr bedingt geeignet, da die Ärzte das einmal aufgenommene Zellbild nicht mehr durchfokussieren können und somit eine für die Befundung wichtige Tiefeninformation nicht zugänglich ist.

Ein Vorteil liegt also darin, dass Zellbild als dreidimensionales Bild aufzunehmen, um so dem Arzt am digital abgespeicherten Bild eine frei einstellbare und jederzeit, auch nachträglich wählbare Fokussierung auf unterschiedliche Fokusebenen zu ermöglichen. Der Arzt muss dabei nicht länger selbst an einem Mikroskop sitzen, sondern der Vorgang kann räumlich und zeitlich unabhängig von der Probe vorgenommen werden. Da das Bild rein digital vorliegt, tritt über die Zeit seit der Aufnahme des Bildes keine Verschlechterung der Daten, im Gegensatz zu den bisher gebräuchlichen Vorgehensweisen, bei denen die archivierten Proben über die Zeit altern und sich in ihrem Zustand verschlechtern. Für jede Nachbefundung würde die Probe wieder in das Mikroskop eingelegt und mit Immersionsöl beaufschlagt. Nach Befundung wird dann die Probe wieder gereinigt, was auch zu Beschädigungen führen kann und insgesamt einen gewissen zeitlichen Aufwand darstellt.

Ein weiterer Vorteil liegt darin, dass wenn sich mehrere Zellen auf einem Bild befinden, der Fokus nacheinander bezüglich jeder einzelnen der jeweiligen abgebildeten Zellen entsprechend eingestellt und variiert werden kann.

In einer weiteren bevorzugten Ausgestaltung des Verfahrens umfasst das Verfahren zum Ermitteln einer zweidimensionalen und/oder dreidimensionalen Information einer Zelle mittels eines Analyzers weiter den Schritt
f1) Zuordnung der Zelle zu einem Zellentyp anhand vorbestimmter Informationen und der ermittelten zweidimensionalen und/oder dreidimensionalen Information der Zelle.

Dies hat den Vorteil, dass eine automatisierte Zuordnung der Zelle zu einem Zelltyp besonders zuverlässig und weniger fehleranfällig vorgenommen werden kann.

In einer weiteren vorteilhaften Ausgestaltung des erfindungsgemäßen Verfahrens zur virtuellen Annotation einer Zelle, von Bestandteilen einer Zelle und/oder eines biologischen Präparats und/oder des Verfahrens zur automatisierten Erkennung, Identifikation und/oder Klassifizierung einer Zelle, von Bestandteilen einer Zelle und/oder eines biologischen Präparats umfasst das Verfahren ein Verfahren zur Zuordnung einer Zelle zu einem Zellentyp, umfassend ein Verfahren zum Ermitteln einer dreidimensionalen Information der Zelle, wobei an einem ersten Ort die Schritte a1) bis d1) ausgeführt werden, und wobei die in Schritt d1) ermittelten Informationen über eine Daten- und/oder Netzwerkverbindung digital an einen zweiten Ort übertragen werden, und wobei am zweiten Ort die Schritte e1) und f1) durchgeführt werden.

Gegenstand der Erfindung ist auch ein entsprechendes Verfahren zur Zuordnung einer Zelle zu einem Zellentyp, wobei jedoch das Ermitteln einer dreidimensionalen Information der Zelle anhand eines sonstigen geeigneten Verfahrens zum Ermitteln einer dreidimensionalen Information der Zelle erfolgt.

Dies hat den Vorteil, dass eine Telemedizin für die Hämatologie ermöglicht wird. Z.B. werden hierdurch konsiliarische Befundungen von Experten an anderen Standorten möglich. Somit kann an einer aufgenommenen Zelle eine Feindiagnose mit Fokussieren auch remote vorgenommen werden. Weiter ermöglicht dies eine Konsultation eines Zweitarztes zur Nach- bzw. Gegenklassifizierung zur Absicherung der Diagnose in der Datenbank. So kann z.B. auch ein Ground-Truth Datensatz mit besonderen und insbesondere seltenen Pathologien entstehen, da sich über die Remote Funktionalität und die Befundung mit 3D Bildern die Ärzte weltweit leichter und effizienter zusammenschließen können

Vorteilhafterweise werden gesicherte Befunde in Cloud-Server-Datensätzen zur automatischen Erweiterung eines Trainingsdatensatzes, insbesondere bei pathologischen Fällen, genutzt.

Vorteilhafterweise wird ein weltweit lernendes System etabliert. Dies ermöglicht es, dass auch für sehr seltene Krankheitsbilder schnellstmöglich größere Datensätze zusammen kommen können. Diese Datensätze können dann vorteilhafterweise auch für automatische Computerlernalgorithmen genutzt werden, so dass letztlich auch für die automatisierte Bewertung und/oder Zuordnung der Zellen eine breitere und abgesicherte Basis verfügbar ist.

Vorteilhafterweise werden auch die Patientendaten in einer Datenbank vorgehalten. Dies hat den Vorteil, dass auch später auftretenden Krankheitsbildern frühere Datensätze zu Blutbildaufnahmen analysiert werden und auf Auffälligkeiten untersucht werden können, so z.B. im Falle der Hämatologie, um sehr frühe Entwicklungsstufen oder Anzeichen einer Leukämie zu entdecken bzw. zu erkennen. Sind diese Daten von einem System gelernt, kann diese Analyse auf den Bildern automatisiert und ohne Mehraufwand ablaufen, da die Zellbilder vorteilhafterweise von Computern vorausgewertet werden.

Bevorzugt erfolgt die Darstellung der Zellen und/oder Proben mittels einer 3D Anzeigeeinrichtungen, bevorzugt z.B. einem autostereoskopischen 3D Display. Dies ermöglicht dem Arzt einen neuartigen 3D Seheindruck der zu untersuchenden Zellen und/oder Proben geben zu können. Insbesondere wird dieser Vorteil auch in der Telemedizin erfindungsgemäß erzielt.

Vorteilhafterweise wird ein kompaktes Datenformat für die Bilddaten aus der Lichtfeldkamera eingesetzt. Bevorzugt werden die Bilddaten komprimiert. Dies ermöglicht es, den benötigten Speicherplatz möglichst klein zu halten, was erst die Speicherung der vielen Patientendatensätze ermöglicht, die dann als Ground Truth z.B. auch für ein computerlernendes System verwendet werden können im Sinne einer automatisierten Medizin und/oder als wertvolles Assistenzsystem für Ärzte.

Vorteilhafterweise erfolgt eine Nutzung von Cloud-Lösungen und/oder Server-Lösungen für die Bildspeicherung und Daten der Befundung und vorteilhafterweise auch für die Sicherung erweiterter Daten, welche Person, z.B. Arzt, wann und auf welchem System mit welcher Konfiguration oder auch welchem Softwarestand die betreffende Zelle befundet hat oder auch wann und wo die betreffende Probe dem Patienten entnommen wurde und Informationen über den Transport in das Labor. Alternativ können diese Informationen bevorzugt auch über eine Verknüpfung mit einem anderen Speichersystem bereitgestellt werden. Vorteilhafterweise wird auch der Zeitpunkt der Befundung entsprechend abgespeichert.

Bevorzugt wird anstelle der Zelle ein medizinisches Präparat entsprechend angefärbt.

Vorteilhafterweise handelt es sich bei den dreidimensionalen Informationen und/oder zweidimensionalen Informationen der Zelle um geometrische Informationen der Struktur der Zelle bzw. des medizinischen Präparats.

Dabei wird unter einer dreidimensionalen Information bzw. einer dreidimensionalen Bildinformation z.B. verstanden, dass das Bild neben der flächenhaften Bildinformation zusätzlich zumindest für einen Bildpunkt auch eine Tiefeninformation in axialer Richtung des Abbildungsstrahlenganges enthält, also z.B. in Z-Richtung, die linear unabhängig von der X- und der Y-Richtung ist. Dabei ist die Tiefeninformation weiter unterteilt und umfasst für den mindestens einen Bildpunkt mindestens zwei, bevorzugt drei, besonders bevorzugt für mindestens 4, unterschiedliche Positionen in der Z-Richtung eine Information. Die Tiefeninformation ist also weiter unterteilt und/oder differenziert. Bevorzugt ist die Tiefeninformation insofern nicht als lediglich summarisch anzusehen. Ein Beispiel für eine dreidimensionale Information bzw. dreidimensionale Bildinformation könnte z.B. eine dreidimensionale Abbildung eines Objektes sein, die auf mindestens zwei unterschiedlichen Flächen liegende Informationen für ein Raster mit Bildpunkte in X-Richtung und Y-Richtung umfasst. Beispielsweise können die mindestens zwei unterschiedlichen Flächen die Vorder- und Rückseite eines zumindest teilweise transparenten Objektes sein, z.B. die Oberflächenpunkte einer Organelle innerhalb einer Zelle oder die äußere Begrenzung der Zelle selber. Besonders bevorzugt ist jede der mindestens zwei unterschiedlichen Flächen zusammenhängend und damit stetig und differenzierbar, so wie es für abgeschlossene Zellbestandteile typischerweise vorausgesetzt werden kann. Alternativ können die unterschiedlichen Flächen auch zwei parallele Ebenen sein, für die die Objektinformation bestimmt wird. Beispielsweise in technischen Ausführungen der Bestimmung der dreidimensionalen Information mit mikroskopischen Systemen können die Flächen bevorzugt normal zur Z-Richtung sein. Bevorzugt sind die Flächen dabei ebene Flächen. Ist das Objekt beispielweise eine Zelle, so könnten die Tiefeninformation im Bild im Bereich des Zellkerns 4 Tiefen-informationen enthalten, die z.B. folgende Abfolge haben mit Rückseite der Zelle, Rückseite des Zellkerns, Vorderseite des Zellkerns und Vorderseite der Zelle. Im Falle komplexerer Strukturen in dem zumindest teilweise transparenten Objekt können auch mehr als 4, sogar deutlich mehr als 4 Tiefeninformationen für einen Bildpunkt vorliegen, wenn mehr als zwei Strukturelemente des Objektes in der Messrichtung vorliegen, deren Abgrenzungen mit den gewählten optischen Methoden kontrastierbar sind.

Dreidimensionale Information umfassen insbesondere auch 2,5-dimentionale Informationen. Dabei wird unter einer 2,5-dimensionalen Information eine topographische Information z.B. in Form einer Höhe und/oder optischen Dicke verstanden. Ein Beispiel für eine 2,5-dimensionale Information könnte z.B. ein Konturbild sein, dass in der z-Richtung die Topografie der Oberfläche des Objektes oder alternativ den Verlauf der optischen Dicke des Objekts wiedergibt. Tiefer im Objekt liegende Strukturen, strukturelle Bereiche oder abgrenzbare Struktureinheiten, wie sie Beispielsweise in Zellen mit Kern und Zytoplasma vorliegen, oder die Information über die Lage bzw. die Form oder Topografie der Rückseite des Objekts können bei der Höheninformation im 2,5-dimensionalen Bild nicht aufgelöst und dargestellt werden. Im Falle der optischen Dicke wird die jeweilige auf einem Lichtweg durch das Objekt summarisch auftretende optische Weglänge als optische Dicke dargestellt. Beispielsweise wird die Summation der optischen Dicke bei einem Interferometer nach Mach-Zehnder- bzw. Michelson Bauart durch Vergleich der optischen Weglängen von Referenz- und Objektstrahlengang mit einem Interferogramm bestimmt, wo aus den auftretenden Interferenzstrukturen dann die optische Dicke für jeden benachbarten Pixel gemäß der lateralen Auflösung des Detektors bestimmt werden kann. Die 2,5-dimensionale Information unterscheidet sich von der 3-dimensionalen Information dadurch, dass für jeden Bildpunkt genau eine Tiefeninformation vorliegt, die die Topografie des Objektes bzw. die optische Dicke beschreiben und z.B. die Topografie des Objektes in der Art eines Reliefbildes wiedergeben.

Dabei wird unter einer zweidimensionalen Information z.B. eine Bildinformation verstanden, die einen Objektbereich in ein flächenhaftes Bild abbildet mit den lateralen Koordinaten in X- und Y-Richtung, die die Fläche beschreiben und aufspannen. Die einzelnen Bildpunkte eines zweidimensionalen Bildes werden auch als Pixel bezeichnet und enthalten neben den Information zu Helligkeit und/oder Farbe und/oder Polarisation keine weiteren Informationen zu einer mechanischen Höhe oder optischen Dicke des Objektes.

Zur eindeutigen Interpretation der Bilddaten mit zweidimensionaler und/oder dreidimensionaler Information ist es im Allgemeinen nötig, dass bekannt ist, in welchem Koordinatensystem, z.B. kartesisch oder zylindrisch, und mit welcher Orientierung der Koordinatenachsen das zugrunde gelegte Koordinatensystem definiert ist.

In weiteren vorteilhaften Ausgestaltungen handelt es sich bei den zweidimensionalen und/oder dreidimensionalen Information um Informationen bezüglich intrazellulärer Strukturen, wie z.B. Zellorganellen und/oder geometrischen Strukturen eines Gewebeschnitts. Informationen bezüglich optischer Pfadlängen entsprechen im Allgemeinen ohne Weiteres keinen geometrischen Informationen und es handelt sich daher bei optischen Pfadlängen diesbezüglich nicht um geometrische Informationen.

Bevorzugt handelt es sich bei dem Färbeprotokoll um das Färbeprotokoll May-Grünwald-Giemsa, Modified-Wright Färbung, Wright-Gimsa Färbung und/oder Romanowsky Färbung und/oder um eine vorbestimmte Farbtabelle, die bevorzugt keinem chemischen Färbeprotokoll entspricht.

Bevorzugt wird bei der digitalen Färbung der Zelle eine Farbverteilung nach einem der oben genannten Färbeprotokolle angewendet. Bevorzugt werden dazu Bilder des technischen Kontrasts aus Schwarz/Weiß und oder gemäß von erweiterten Kontrastverfahren mittels durch Computerlernen bestimmter Färbeeigenschaften umgefärbt. Dazu werden bevorzugt 3D und/oder volumetrische Informationen verwendet, da dies vorteilhaft gegenüber der Verwendung eines reinen 2D Farbmappings ist.

Vorteilhafterweise handelt es ich bei dem Färbeprotokoll um ein künstliches Farbmodell, das z.B. bestimmte kritische Merkmale betont, um Ärzten die Befundung zu vereinfachen oder etwa unerfahreneren Ärzten die Diagnose überhaupt erst zu Ermöglichen. Das künstliche Farbmodell kann dabei ähnlich einer Falschfarbendarstellung für technische Bilder ausgestaltet sein, z.B. ähnlich wie bei Wärme- oder IR-bildern, die als Farbbild dargestellt werden. So können auch innerhalb der Befundung ein und derselbe Zelle einfach die Färbemodelle gewechselt werden, was bei einer klassischen chemischen Färbung nicht möglich wäre.

Bevorzugt werden Zellen, die unterschiedlichen Klassen zugeordnet sind, unterschiedlich digital gefärbt. Vorteilhafterweise werden alle Zellen im Bild, die aktuell nicht betrachtet werden sollen, ausgeblendet. Bevorzugt wird alternativ nur eine ausgewählte Menge von unterschiedlichen Zellgruppen dargestellt.

Bevorzugt werden 3D Informationen der Zellen, ggf. auch in Kombination mit den erweiterten Kontrastierungsmethoden wie Phasenkontrast oder DIC, genutzt, um die wegfallende Farbinformation der entfallenden unmittelbaren Färbung durch die 3D Information zu ersetzen. Dies ermöglicht es, die entsprechend gelernte Information bezüglich des ungefärbten Bildes möglichst robust und weniger fehleranfällig in die Farbinformation umzusetzen.

Bevorzugt wird die vorbestimmte Zuordnung zwischen den zweidimensionalen und/oder dreidimensionalen Informationen der Zelle und einer Färbung einer entsprechenden Zelle und/oder einer Struktur innerhalb der Zelle mittels eines Färbeprotokolls z.B. wie folgt in einer Lernphase ermittelt.

Hierzu wird vorteilhafterweise zunächst die Zelle oder werden die Zellen ohne Färbung in einem ersten Bild abgebildet. Anschließen erfolgt eine Färbung der Zellen gemäß des jeweilig gewünschten Färbeprotokolls. Dann wird die gefärbte Zelle oder werden die gefärbten Zellen in einem zweiten Bild abgebildet. In dem ersten und zweiten Bild wird dann die Zelle (bzw. die Zellen) erkannt und segmentiert und die ungefärbten und gefärbten Zellen werden jeweils entsprechend zugeordnet.

Vorteilhafterweise werden die Zellen nach Zelltyp gruppiert, z.B. rote Blutzellen (RBC), weiße Blutzellen (WBC) (ggf. inklusiv 5 Part Diff.) und/oder klassifiziert, z.B. mittels Computerlernen und/oder neuronalen Netzen.

Im nächsten Schritt erfolgt das Computerlernen der Charakteristika der Färbung für jede der Zellgruppierungen getrennt voneinander und es wird anschließend eine spezifische digitale Färbevorschrift für die ungefärbten Zellen, vorteilhafterweise separat für jede Zellgruppierung, erstellt.

Zur Verifizierung der digitalen Färbevorschrift erfolgen dann eine Anwendung der Färbung auf die ungefärbten Zellen und ein Vergleich mit einer Bewertung des Ergebnisses der digitalen Färbung mit der tatsächlich gefärbten Probe, wobei ein möglichst gleiches Aussehen der gefärbten und digital gefärbten Zellen angestrebt wird. Vorteilhafterweise erfolgt dann eine Bewertung des Ergebnisses gemäß vorgebestimmten Qualitätskriterien und/oder nach maximal erlaubten Restabweichungen. Wegen der in der Medizin immer sehr kritischen Frage der Farbechtheit können die Farbabweichungen auch gegen einen externen Bezugsstandard bewertet werden, wie er z.B. in DICOM für Displays und Anzeigegeräte beschrieben ist. Da der Standard nicht für diesen Anwendungsfall geschrieben ist, können die Bewertungen ggf. nur sinngemäß übertragen und angewendet werden. Das Färbemodell wird entsprechend für jedes Färbeprotokoll und jede Zellgruppierung individuell bzw. gezielt erstellt, überprüft und abgespeichert.

Bevorzugt umfasst die Erstellung der Zuordnung Anwendung von Methoden des maschinellen Lernens und/oder künstlicher Intelligenz (KI) mittels eines Computers. Dies hat den Vorteil, dass so eine weitere Optimierung der Farbdarstellung erreicht werden kann.

Zur Optimierung der Farbdarstellung kann die Färbung vorteilhafterweise also auch mit Methoden des maschinellen Lernens und/oder der künstlichen Intelligenz (KI) bestimmt werden. Beispielsweise kann diesbezüglich vorteilhafterweise wie folgt vorgegangen werden. Um dafür ein Computersystem zu trainieren, werden z.B. Proben in bisher üblicher Art auf einem Objektträger aufgebracht. Dann wird zunächst die noch ungefärbte Probe mit dem optischen System mit technischem Kontrast vermessen und die Bilddaten gespeichert. Hier ist es vorteilhaft, wenn die Bilddaten auch die lateralen Ortsinformationen der Probe erhalten bzw. die laterale Zuordnung von Teilbildern bei der Erfassung größerer Probenbereiche erhalten bleibt. Anschließend wird die Probe z.B. nach einem der bekannten typischen Färbeprotokolle angefärbt. Danach wird die Probe dann erneut optisch erfasst. Diesmal insbesondere als Farbbild, um die Färbung für jeden Probenbereich mit einem Farbwert und/oder einer Gruppe von Farbwerten (z.B. RGB) zu beschreiben. Das Farbbild bzw. die Vielzahl von Teilbildern der Probe dienen dann als Referenz für die Einfärbung jeweiliger Zellbereiche bzw. Zellstrukturen mit der jeweiligen Färbung. Ergänzend kann die Probe vorteilhafterweise auch mit anderen technischen Kontrasten erfasst werden, um z.B. Übergänge in den Zellstrukturen besser erkennen oder kontrastieren zu können, als dies allein mit der Färbung möglich wäre. Nun wird ein zum Lernen ausgebildetes Computersystem mit den Datensätzen von ungefärbter Probe und gefärbter Probe mit einer Vielzahl von Teilbildern und/oder einer Vielzahl von Proben gespeist. Das System soll dabei entsprechende Zellbereiche bzw. Bildstrukturen lernen, insbesondere wie der technische Kontrast bzw. die Bildstruktur im technischen Kontrast und im Farbkontrast der jeweiligen Färbung aussieht. Hierbei ist es vorteilhaft, wenn die räumliche Zuordnung von Bildpunkten bzw. Bildbereichen von den Bildern mit technischem Kontrast und den Farbbildern bekannt sind. Dazu ist die Kenntnis der zumindest lateralen Bildkoordinaten von großem Vorteil und erleichtert die Zuordnung, da nur eine Verschiebung und/oder gegebenenfalls Rotation der Bilder (z.B. Teilbilder und/oder durch Stitching gefügten Gesamtbilder) bestimmt werden muss. Solche Zuordnungen können beispielsweise mit Korrelationen der Bilder bzw. von Bildbereichen bestimmt werden.

Alternativ kann auch eine "künstliche" Farbtabelle erstellt und verwendet werden, die vorteilhafterweise z.B. so optimiert ist, dass unterschiedliche Bestandteile von Zelle und/oder Gewebe mit möglichst großem Farbkontrast dargestellt werden oder für Zellstrukturen, die für eine Zell-Entwicklungslinie charakteristisch sind, dann ähnliche Farben bzw. Farbtöne verwendet werden. Hier wird also vorteilhafterweise einer bestimmten Art von Zellstruktur oder Zelle dann ein Farbwert zugeordnet und die Farbsättigung dann z.B. über die Dicke der jeweiligen Zellstruktur entsprechend gewählt.

Bevorzugt umfasst das Verfahren zum Trainieren eines Verfahrens zur digitalen Anfärbung einer Zelle und/oder eines medizinischen Präparats weiter die oben aufgeführten Schritte eines Verfahrens zum Ermitteln einer dreidimensionalen Information einer Zelle mittels eines Analyzers entsprechend eines vorteilhaften erfindungsgemäßen Verfahrens zur digitalen Anfärbung einer Zelle und/oder eines medizinischen Präparats.

Zur digitalen Anfärbung einer Zelle wird bevorzugt z.B. wie folgt vorgegangen. Zunächst wird das gewünschte Färbeprotokoll ausgewählt und die ungefärbten Zellen werden in einem ersten Bild abgebildet. Die Zelle oder die Zellen werden in dem ersten Bild segmentiert. Dann erfolgen eine Zuordnung der Zellen zu Zellgruppierung und/oder eine Bestimmung des Zelltyps für die Auswahl eines zellspezifischen Färbemodells. Im nächsten Schritt wird die Zelle oder werden die Zellen angefärbt. Anschließend erfolgt die Anzeige eines digital gefärbten Bildes, z.B. eines Blutausstrichs, auf einer Ausgabeeinheit.

Vorteilhafterweise wird dasselbe ungefärbte Bild in der passenden, jeweils regional üblichen Färbung eingefärbt. Dies ermöglicht die gemeinsame Befundung einer Probe durch Experten, die an voneinander abweichende Färbungen gewöhnt sind.

Auch ist ein Austausch des jeweiligen Färbeprotokolls jederzeit möglich, unabhängig davon, ob die ursprüngliche Blutprobe noch vorhanden ist, zum z.B. weitere Blutausstriche zu machen. Ein weiterer großer Vorteil beim digitalen Umfärben ist, dass ein und dieselbe Zelle unterschiedlich gefärbt werden kann, was bei einer chemischen Färbung der realen Zelle nicht möglich ist. Dies ermöglicht z.B. auch die regional übergreifende Konsultation über bisherige Grenzen von Färbeprotokollanwendungen hinaus. Aufgrund der vollständig digital vorliegenden Information erübrigen sich bisherige Problemfelder, wie z.B. ausbleichen der Probe über die Zeit.

Da ein Bild mit einem technischem Kontrast also vorteilhafterweise mit unterschiedlichen Farbtabellen eingefärbt werden kann, können so auch Mediziner aus unterschiedlichen geografischen Regionen, in denen unterschiedliche Färbeprotokolle zum Einsatz kommen, dann an ein und derselben Probe eine Befundung vornehmen.

Durch den Einsatz von generischen oder künstlichen Farbtabellen können weiter auch kontrastreichere Bilddarstellungen gewonnen werden, die so über Färben nicht möglich wären.

Weiter wird ermöglicht, dass bei etwaigen Nachuntersuchungen zu spezifischen Patienten auch Färbeprotokolle anwendbar werden, die zum Zeitpunkt der ursprünglichen Befundung noch nicht bekannt oder nicht verfügbar waren. Diese ermöglicht es, dass der medizinische Fortschritt bezüglich des Standard of Care auch für bestehende Proben bzw. deren Bilder anwendbar ist und zu entsprechend qualitativ höherwertigen Befundungen führen kann.

Bisher wurden die Blutausstriche in einem vorgelagerten, aufwändigen Schritt angefärbt, um die Zellen im Blutausstrich für das Mikroskop und die Beobachtung durch den Arzt hinreichend gut sichtbar zu machen, um sie mit der hochauflösenden optischen Mikroskopie analysieren zu können. Diese Methode ist seit vielen Jahrzehnten gebräuchlich und etabliert. Weltweit sind diesbezüglich verschiedene Färbeprotokolle, wie z.B. May-Grünwald-Giemsa, Modified-Wright-Stain, Wright-Giemsa Stain, Romanowsky Stain etabliert, wobei sich selbst innerhalb der Protokolle laborspezifische Unterschiede ergeben können, da manche Labore die Färbung geringfügig nach ihren Wünschen adaptieren. Dies führt dazu, dass die Vergleichbarkeit der Analyse von Blutausstrichen nur begrenzt möglich ist, insbesondere da in der automatisierten Analyse und Klassifikation von Blutausstrichen nur Zellen gut miteinander verglichen werden können, die nach demselben Protokoll angefärbt worden sind. Ein weiterer Nachteil der bisherigen Vorgehensweise ist, dass der Farbstoff eine Einwirkzeit auf die ausgestrichene Blutprobe von etwa 10 - 15 Minuten benötigt. Dies kann bei Analysen zu akuten Krankheitsbildern sehr nachteilig sein.

Bevorzugt sollte möglichst vermieden werden, der Probe Farbstoff zur Anfärbung der Zelle zuzuführen, wenigstens bevor diese einmal abgebildet wurde.

Bevorzugt können erfindungsgemäß mit der neuen digitalen Anfärbung für unterschiedliche Zelltypen auch unterschiedliche Färbeprotokolle ausgewählt werden, weil z.B. deren Spezifika für den jeweiligen Zelltyp besonders gut passen. So könnte ein Arzt bzw. Hämatologe oder Pathologe persönliche Färbeschemata erstellen, wo er einem Zelltyp eine bestimmte Färbung zuordnet und die bisherige Einschränkung von nur einer Färbung für ein Slide komplett durchbrochen und aufgehoben wird. In Erweiterung dieser "individuellen" Färbung können vorteilhafterweise auch generische digitale Färbeprotokolle entstehen, die zur besseren Erkennbarkeit von Merkmalen und Strukturen den technischen Farbraum weiter nutzen. Die chemische Färbung arbeitet, in jedem Färbeschritt typischerweise, im Wesentlichen über einen Farbstoff, der sich in unterschiedlichen Mengen an geeignete bindungsfähige Zellstrukturen anlagert. Je mehr Farbstoff sich anlagert, umso mehr Licht wird aus dem Spektrum des Beleuchtungslichtes gemäß des Absorptionsspektrums des jeweiligen Farbstoffes heraus absorbiert. Das Transmissionsbild enthält so eine höhere Farbsättigung (S) für den/die Farbwert/e des Farbstoffs (V). Werden mehrere Farbstoffe in einem Färbeprotokoll gleichzeitig oder seriell angewendet, die sich an unterschiedlichen Stellen der Zellstruktur anbinden, so überlagern sich die Effekte. Jeder Farbstoff verändert für seine spezifischen Farbwerte V je nach Menge des angelagerten Farbstoffs dann den Sättigungswert S. Über die Zahl Farbstoffe und deren spezifischen spektralen Charakteristika ist die Zahl der veränderten Farbwerte V begrenzt. Typische Färbungen in der Medizin liegen im rot-blauen Bereich, so dass z.B. Farbwerte im grünen oder gelben Spektralbereich nahezu unverändert bleiben. Die digitale Färbung kann vorteilhafterweise sowohl mir schärferer Farbtrennung als auch mit breiterer Abdeckung im optischen Spektrum arbeiten.

Bei manchen Untersuchungen kann es jedoch vorteilhaft sein, nicht völlig auf die Zugabe von Farbstoffen und/oder pharmazeutischen und/oder chemischen Zusatzstoffen zu verzichten. Z.B. bei Reticulozyten hat der Farbstoff neben der reinen Farbwirkung noch den gewünschten Nebeneffekt, dass die in der Zelle noch verfügbare RNA verklumpt und erst dadurch im Mikroskop abbildbar und kontrasttierbar wird. Die unverklumpte RNA ist von der Dimension her kleiner als es mit herkömmlichen optischen Mikroskopen auflösbar wäre.

Vorteilhafte Ausgestaltungen des erfindungsgemäßen Verfahrens basieren auf einem optischen Mikroskop, das vorteilhafterweise zur Bildaufnahme mit erweiterten optischen Kontrastierungsmethoden ausgestattet ist. Diese erweiterten Kontrastierungsmethoden können beispielsweise umfassen Phasenkontrast (PC), Differential-Interferenzkontrast (DIC), Polarisationskontrast (POL), Phasenkontrast (z.B. nach F. Zernike), spektraler Kontrast, Absorptionskontrast (z.B. wie bei der multispektralen oder auch hyperspektralen Bildgebung), Autofluoreszenzkontrast, Interferometrie (bevorzugt in der Ausführung als digitales holografisches Mikroskop (DHM)), induzierbare Doppelbrechung bzw. optische Aktivität (z.B. durch Anlegen äußerer Felder wie z.B. elektromagnetischer oder statischer elektrischer oder magnetischer Felder hervorgerufen, wenn z.B. Symmetrieeigenschaften von Substanzen in den Zellen oder den Zellen aufgehoben werden), Hyperspectral Imaging (Reiner Farbkontrast in einem erweiterten Spektralbereich, z.B. UV, VIS, NIR, MIR und/oder FIR oder ausgewählten Teilbereichen daraus), und/oder Strukturierte Beleuchtung (z.B. mit vorbestimmten Intensitätsverteilungen und/oder Phasenverteilungen, bevorzugt in der Pupillenebene eingestellt).

Bevorzugt sind unterschiedliche Kontrastierungsmethoden an einem Mikroskopiersystem parallel vorhanden, insbesondere, wenn diese z.B. untereinander wechselwirkungsfrei nutzbar sind und/oder rückwirkungsfrei sind. Andernfalls werden die unterschiedlichen Kontrastierungsmethoden bevorzugt zeitlich nacheinander ausgeführt. Kommt mehr als eine Kontrastierungsmethode an einer Probe bzw. in einem Bereich einer Probe zum Einsatz, kann das
allgemein auch als multi-modale Bildgebung beschrieben werden, da jede der Kontrastierungsmethoden für sich als eine Modalität aufgefasst werden kann.

Alternativ können die Methoden vorteilhafterweise auch im Sinne einer multifaktoriellen bzw. vollfaktoriellen Versuchsplanung miteinander verkoppelt werden, um die Zahl der benötigten Bildaufnahmen zu reduzieren. Beispielsweise wird ein Spektralkontrast in unterschiedlichen Wellenlängenbereichen gemessen und in diesen dann parallel auch DIC, Polarisationskontrast oder Autofluoreszenzkontrast parallel mit angewendet. Oder beispielsweise wird in einem spektralen Bereich ein Polarisationskontrast angewendet und in einem anderen DIC. Aus den aufgenommenen Teilbildern können dann Gesamtbilder synthetisiert werden, um ein Abbild der Probe mit technischem Kontrast zu erhalten, dass einen möglichst großen Informationsgehalt in Bezug auf die Differenzierung von Eigenschaften der Probe bzw. von Teilen der Probe aufweist.

Werden die unterschiedlichen Kontrastierungsmethoden zeitlich nacheinander ausgeführt, so ist erfindungsgemäß bevorzugt ein particel image velocimetrie (PIF) zur Verfolgung der jeweiligen Trajektorien vorgesehen, da sich bei einer Kombination mit einer Mikrofluidik zwischen den Bildern die Zellen bewegen. Dies ermöglicht, die in den unterschiedlichen Modalitäten gewonnenen Farbinformationen dann wieder für jede Zelle korrekt zusammen bringen zu können. Bevorzugt umfasst die PIV auch die Rotation der Zelle und vorteilhafterweise auch die Defokussierung.

Über die erweiterten Kontrastierungsmethoden bekommen die Proben z.B. einen Farbkontrast aus Interferenzen, beispielweise DIC oder Polarisation, der dann im digitalen Bild für eine gute Auflösung der Strukturen in der Zelle sorgt und/oder eine gute Kontrastierung für die Zelle ermöglicht.

Erfindungsgemäß ist es besonders vorteilhaft, wenn neben der Amplituden- bzw. Intensitätsinformation und möglicherweise auch einer Farbinformation zusätzlich eine 3D Information vorliegt. Die Bilder der Lichtfeldkamera für die 3D Aufnahme können auch als volumetrische und/oder tomografische Daten vorliegen. Diese 3D Information erlaubt erfindungsgemäß auch ein nachträgliches rein digitales Durchfokussieren am aufgenommenen Bild. Dies ist für die Überprüfung der automatischen Klassifizierung durch den Arzt und für die spezielle und sichere Beurteilung pathologischer oder auffälliger Zellen in diesem Zusammenhang von besonderem Vorteil.
Bevorzugt sind erfindungsgemäß im digitalen Färben dann auch neue Färbemodi vorgesehen, wo die aktuell angezeigte Färbung dann nur von dem Bereich der Zelle abhängt, der z.B. unterhalb der Fokusebene liegt. So kann die 3D Tiefenwahrnehmung des Arztes weiter verbessert werden. Alternativ können auch nur Bereiche oberhalb der aktuellen Fokusebene verwendet werden oder aber Bereiche in einer gewissen einstellbaren Schichtdicke um die konkrete Fokusebene herum.

Für die erfindungsgemäße digitale Anfärbung wird also bevorzugt ein mikroskopisch mit einem Analyzer aufgenommenes Bild dann so nachbearbeitet, dass es so aussieht, wie es wahrscheinlich ausgesehen hätte, wenn es mit einem klassischen Mikroskop von gefärbten Zellen aufgenommen worden wäre. Zur Umsetzung wird für jedes Färbeprotokoll eine gewisse Zahl an ungefärbten Proben mit einer Vielzahl von Zellen in dem neuen System aufgenommen. Die Zellen können für den Vergleich manuell oder auch automatisch im Bild erkannt und segmentiert werden. Danach werden dieselben Proben mit dem gewünschten Färbeprotokoll gefärbt und anschließend mit
einem Mikroskop dieselben Zellen erneut aufgenommen. Dabei ist es wichtig, dass das Mikroskop über eine gute Farbtreue und eine Farbkamera verfügt, besonders bevorzugt über eine für möglichst farbechte Bildaufnahme optimierte Farbkamera, die gegebenenfalls auch mit speziellen Proben oder Kalibrierabläufen kalibriert wurde, z.B. gemäß des DICOM Standard in der Medizin.

Typische Farbkameras haben ein Bayer Pattern, bei dem 50 Prozent der Pixel überwiegend für grün, 25 Prozent der Pixel überwiegend für Blau und die verbleibenden 25 Prozent der Pixel überwiegend für Rot empfindlich sind. Um eine vollständige Farbinformation zu den RGB Werten für jedes Pixel zu erhalten, werden dann z.B. für den zu bestimmenden Farbwert die Farbwerte von den Nachbarpixeln der entsprechenden Farbe interpoliert. Gegebenenfalls werden dazu noch Stetigkeitsinformationen aus den Pixeln anderer Farbwerte mit betrachtet und herangezogen. Bevorzugte Farbkameras sind z.B. 3-Chip Farbkameras, die für jeden Bildpunkt unmittelbar einen Intensitätswert für Rot, Grün und Blau parallel messen. Diese bevorzugten Farbkameras sollen also Kameras sein, die für jeden Pixel die benötigten Farbwerte unmittelbar messen.

Da nun für jede Zelle die Information aus ungefärbter Zelle und gefärbter Zelle vorliegt, kann die Farbinformation, bevorzugt z.B. über Methoden des Computerlernens, zwischen den beiden Proben verknüpft werden. Bevorzugt erfolgt diese Verknüpfung für jede Klasse von Zellen getrennt, um spezifisches Färbeverhalten möglichst gut zu erfassen. Alternativ ist es vorteilhaft, zur Vereinfachung des Aufwands und Lernumfangs, gleiche Zelltypen, die sich z.B. in unterschiedlichen Entwicklungsstufen befinden, gemeinsam zu lernen. Dies könnte vorteilhafterweise z.B. bei roten Blutkörperchen erfolgen, die in unterschiedlichen Alterungszuständen vorliegen, wo sie die geometrische Form leicht ändern. Ebenso ist es vorteilhaft möglich, bei weißen Blutkörperchen die 5 Hauptgruppen, die in der 5 Part Differentialdiagnostik unterschieden werden, gemeinsam zu trainieren. Es kann dann vorteilhaft sein, innerhalb einer solchen Gruppe in einem zweiten Schritt noch die Färbungen spezifischer Merkmale zu lernen, um eine möglichst gute und umfassende Farbkontrastierung zu erhalten.

Besonders bevorzugt wird bei dem Lernen die 3D Information, z.B. in Form von topografischer Information oder als volumetrische bzw. tomografische Information verarbeitet. Für das Lernen kann diese 3D Information und vorteilhafterweise daraus abgeleitete Information, z.B. eines effektiven 3D Profils, sowohl für das ungefärbte Bild als auch bevorzugt für das gefärbte Bild, verwendet werden. Für das Lernen sind dann alle vom Computerlernen bekannten Verfahren prinzipiell anwendbar, wie z.B. PLS, PLSDA, PCA oder auch neuronale Netze (CNN) oder deep CNNs.

Für ein gutes Lernergebnis und damit eine möglichst realistische digitale Färbung erreicht werden kann, ist es vorteilhaft, wenn pro zu lernendem Zelltyp und/oder pro Gruppe von gemeinsam gelernten Zellen die Klassifizierung in mindestens so vielen Farbwerten erfolgt, wie an der Originalprobe unterscheidbar und/oder auf der Anzeigeeinrichtung darstellbar sind. Bei Computern sind für die Farbdarstellung Farbräume gebräuchlich, wo für jeden Bildpunkt pro Farbe z.B. 256 Farbwerte, also 8 bit bzw. 1 Byte Farbtiefe, oder 65536 Farbwerte, also 16 bit bzw. 2 Byte bzw. 1 double Byte verwendet werden.

Um effizient Datenvolumen zu sparen, kann es vorteilhaft sein, eine andere Darstellung des Farbraumes und/oder der Farbwerte als RGB zu wählen. Da sich die effektiven Farbwerte zwischen den Zelltypen unterscheiden können und idealisiert innerhalb eines Zelltyps sich nur in der Sättigung bzw. Helligkeit unterscheiden, wird vorteilhafterweise ein Farbwert und ein Helligkeitswert gespeichert. Dies hat den Vorteil, dass nicht für jeden Bildpunkt die volle Farbinformation in RGB gespeichert werden muss. Diese vorteilhafte Darstellung wäre z.B. mit einer HSV oder HSL Farbdarstellung vergleichbar. Aufgrund der häufig vergleichsweise ähnlichen Farben in den Bildern ist das Potential zum Sparen von Speicherplatz und/oder Datenvolumen hier besonders hoch.

Ein großer Vorteil des erfindungsgemäßen digitalen Färbens besteht darin, dass Proben nun auch zwischen Regionen verglichen werden können, die typischerweise mit anderen Färbeprotokollen und damit anderer farblicher Wirkung der Zellen arbeiten. Wenn eine zu untersuchende ungefärbte Probe aufgenommen wurde, kann diese erfindungsgemäß wahlweise mit unterschiedlichen gelernten Farbstrukturen eingefärbt werden, die dem jeweiligen regionalen Färbeprotokoll entsprechen. Das bietet zum einen den Vorteil, dass man für unterschiedliche Pathologien nun das bevorzugte oder vorteilhafte Färbeprotokoll verwenden kann. Andererseits kann so auch von einem Arzt ein anderer Arzt für eine Konsiliarmeinung befragt werden, da er ihm nun das Bild in der für ihn gewohnten Färbung zeigen kann. Durch das digitale Färben können die von den Färbeprotokollen entstandenen Hürden umgangen werden, weil jetzt jeder Arzt seine für ihn gewohnte Färbung zur Durchführung der Befundung wählen kann. So können dann auch z.B. Ärzte aus Europa und den USA zum Wohle des Patienten besser zusammen arbeiten. Dies ermöglicht somit eine weltweite Zusammenarbeit der Ärzte und/oder Hämatologen im Sinne der Telemedizin.

Somit kann für die Telemedizin, beziehungsweise über die Telemedizin, mit den digital passend gefärbten 3D Bildern und/oder Datensätzen dann jeder Arzt weltweit zu einer gesicherten Befundung beitragen. Somit kann beispielsweise über eine Datenbank insbesondere für seltene pathologische Fälle an beobachteten und/oder vermessenen Zellen über die weltweite Vernetzung mit der Telemedizin vorteilhafterweise ein sicher bewerteter "Ground Truth" Datensatz entstehen, der eine Kombination aus Bild und/oder 3D Information und der von mindestens zwei Ärzten abgesicherten Befundung besteht. Ein wichtiger Baustein für dieses Vorgehen liegt auch in der erfindungsgemäßen Möglichkeit, im digitalen Datensatz für jede aufgenommene Zelle getrennt und angepasst die Bilder im Fokus verändern zu können, ohne dass der befundende Arzt selber an einem Mikroskop sitz.

Dieser von mehreren Ärzten unabhängig befundete Datensatz kann dann, eine Übereinstimmung der Befundung vorausgesetzt, z.B. zum weiteren Trainieren von Computermodellen verwendet werden, die dann wieder auf die weltweit installierten Geräte zurück gespielt werden können, um damit jedes Gerät in der Erkennungsgüte für bestimmte Zellen, oder in der Fähigkeit, besondere Pathologien sicher automatisiert erkennen zu können, weiter zu entwickeln.

In weiteren vorteilhaften Ausgestaltungen des erfindungsgemäßen Verfahrens kann es auch vorteilhaft sein, dass Analyzer Bilder aufnehmen und diese dann z.B. in eine cloudbasierte Anwendung oder allgemeiner an eine andere Recheneinheit übertragen werden, wo dann die Erkennung der Zellen, deren Segmentierung und eine automatisierte Auswertung, z.B. im Sinne einer Zuordnung zu einer Klasse von Zelltyp oder einer Befundung als Erkennung von bestimmten Krankheitsbildern und/oder die Feststellung eines Verdachtes auf ein bestimmtes Krankheitsbild stattfindet.

Für die Anzeige der aufgenommenen Bilddaten zu einer Zelle gibt es für den Arzt unterschiedliche Möglichkeiten.

Vorteilhafterweise kann z.B. ein herkömmlicher 2D Monitor, ein mobiles Endgerät und/oder ein Tablett PC verwendet werden. Vorteilhafterweise ist die Farbdarstellung so abgeglichen, dass der Arzt von einer farbechten Farbwiedergabe ausgehen kann. Das ist bei der Hämatologie besonders wichtig, weil insbesondere bei pathologischen oder auffälligen Zellen kleine Farbunterschiede und Strukturunterschiede im Bild der Zelle einen Hinweis auf eine Auffälligkeit geben können. Vorteilhafterweise genügt die Farbwiedergabe, insbesondere bezüglich der stabilen Farbwiedergabe z.B. dem DICOM Standard für medizinische Geräte (dicom.nema.org/).

Vorteilhafterweise kann der Anwender und/oder Nutzer dann durch Bedienung an einer Software durch die Zellbilder und/oder das flächige Bild vom Objektträger oder von der Flowcell hindurch fokussieren. Vorteilhafterweise liegt hierfür eine entsprechend sichere, beziehungsweise etwaigen Vorgaben entsprechende Datenverbindung zu den Daten auf einem lokalen Datenspeicher, einem übergeordneten Datenspeicher, z.B. in einem Krankenhaus, und/oder einer Cloud vor. Bevorzugt können für z.B. eine Konsiliaruntersuchung die Daten einem Arzt unmittelbar zugänglich gemacht werden.

Bevorzugt wird ein 3D fähiges Display verwendet, das entweder mit oder besonders bevorzugt ohne weitere optische Hilfsmittel angeschaut werden kann und für den Anwender einen 3D Seheindruck erzeugt. Bevorzugt ist das 3D fähige Display Teil eines Computers und/oder eines mobilen Endgeräts, z.B. einem Tablett. Bevorzugt kann der 3D Effekt dabei ein- und ausgeschaltet werden.

Bevorzugt wird eine Datenbrille, auch als Smart Glasses oder VR-Brille bezeichnet, für die 3D Darstellung des Datensatzes verwendet.

Bevorzugt ist erfindungsgemäß für eine Remote-Befundung in der Telemedizin ein Arbeitsmodus Master-Slave vorgesehen, wenn bei gleichzeitiger Befundung der Bilder durch mehrere Ärzte, z.B. in einer medizinischen Konferenz, einer als Master die Navigation über die Bilder der Zellen und/oder vom Objektträger und/oder der Durchflusszelle (Flowcell) führt und die weiteren Ärzte diesem dann ohne eigenes Steuern des Systems folgen können.

Bevorzugt kann jeder der Ärzte sein Bild auch unabhängig von den anderen durchfokussieren und/oder das Bild nach seinem gewohnten Färbeprotokoll einfärben.

Ein weiterer Gegenstand der Erfindung ist ein digitales System zur Erstellung und/oder Darstellung von Bildern von Zellen und/oder Gewebe umfassend eines oder mehrere der erfindungsgemäßen Verfahren.

**Gegenstände der Erfindung werden im Folgenden noch einmal durch konkrete Ausführungsbeispiele und eine beigefügte Zeichnung näher erläutert. Die Beispiele stellen jeweils bevorzugte Ausführungsformen der Erfindung dar.**

Ein vorteilhafter Verfahrensablauf zum Trainieren eines erfindungsgemäßen Verfahrens zur automatisierten Erkennung, Identifikation, Klassifizierung und/oder virtuellen Annotation einer Zelle, von Bestandteilen einer Zelle und/oder eines biologischen Präparats umfasst z.B. die folgenden Schritte.

Zunächst wird ein 2D und/oder 3D Bild der Probe mit mindestens einem technischem Kontrast bzw. einer Modalität aufgenommen. Die Bildaufnahme erfolgt dabei mittels eines Bildaufnahmesystems für mindestens einen technischen Kontrast bzw. eine Modalität in zwei bzw. drei Dimensionen. Das Bildaufnahmesystem ist zur Bildaufnahme mit unterschiedlichen Modalitäten ausgebildet, die bevorzugt auch parallel betrieben werden können oder automatisch umgeschaltet werden können.

Es erfolgt eine Einfärbung der Zelle und/oder des medizinischen Präparats mittels Durchführung eines Färbeprotokolls, wobei das Färbeprotokoll eine selektive Färbung von vorbestimmten Merkmalen der Zelle und/oder des medizinischen Präparats umfasst und wobei die Färbung eine selektiv schaltbare Färbung umfasst, die Färbung umfassend PE-Vio 770, DAPI (4',6-Diamidino-2-Phenylindole), einen Oberflächenmarker für Lymphozyten, einem Peroxisomenmarker und/oder Concavalin A.

Mittels eines Aufnahmesystem für Referenzbilder, welche nicht notwendigerweise zugleich auch das Bildaufnahmesystem sein kann, werden Referenzbilder der Probe aufgenommen, z.B. mittels Fluoreszenzmikroskopie, FRET oder Facs, nachdem die Probe entsprechend eingefärbt wurde. Die Aufnahme der Referenzbilder erfolgt dabei für unterschiedliche Schaltzustände der selektiv schaltbaren Färbung, z.B. unter Nutzung verschiedener Wellenlängen oder Wellenlängenbereichen.

Die über die Farbstoffe aufgenommen Bilder dienen als Ground Truth und somit als Referenz für die Bilder mit dem technischen Kontrast. Da verschiedene Farbstoffe und/oder Anregungswellenlängen bzw. Anregungsenergien zur Anwendung kommen, wird die Vielzahl von solchen Referenzen zu einem Bild auch summarisch als Referenzbild bezeichnet. Dabei ist bekannt, welche dieser Referenzen mit welcher Abbildungsmodalität entstanden ist bzw. aufgenommen wurde und für welchen Bestandteil im Bild aufgrund der verwendeten Abbildungsmodalität diese Bild dann eine Referenz ist. Für diesen Bestandteil im Bild stellt das Bild nun die neue technische ground truth dar. Folglich ist das Referenzbild dann die Grond Truth für alle in der Zelle über den Einsatz von fluoreszierenden Farbstoffen bestimmbaren bzw. unterscheidbaren Bestandteile.

Diese Referenzbilder, zusammen mit der Information zur jeweiligen Modalität, sind von Menschen, insbesondere Biologen oder Medizinern oder Biochemikern, in gewohnter Art interpretierbar. Es werden von einer Vielzahl von Proben solche technisch kontrastierten Bilder mit 2D und/oder 3D Informationen und die zugehörigen Referenzbilder aufgenommen. Die Bilder werden über die reine Geometrie der Probe über z.B. Referenzmarken räumlich zugeordnet. Alternativ kann über eindeutige Anordnungen von Bildinhalten eine Zuordnung im Sinne einer Registrierung hergestellt werden. So lassen sich die Bildinformationen miteinander verknüpfen und so z.B. verwenden, um computergestützte automatisierte Systeme, die auf Methoden der künstlichen Intelligenz (KI), der artificial Intelligence (AI), z.B. unter Verwendung von neuronalen Netzen (CNN und/oder deep CNN) oder auch bionischen Algorithmen zur Auswertung von bildbasierten und ggf. multimodalen Datensätzen beruhen, zu trainieren, die Unterscheidung der unterschiedlichen Zellstrukturen künftig alleine auf Grundlage der technische kontrastierten Bilder treffen zu können.

Es erfolgt also eine Räumliche Zuordnung und/oder Registrierung von technisch kontrastierten Bildern und Referenzbildern auf Basis von z.B. strukturellen Informationen in den Bildern und der erweiterten Informationen in den Referenzbildern.

Weiter erfolgt ein Anlernen eines computerbasierten Systems zur Unterscheidung von Zellstrukturen bzw. Probenstrukturen alleine anhand der Bilder mit technischem Kontrast. Dabei kann optional, jedoch vorteilhaft, die Durchführung einer Segmentierung des Bildes zum Erkennen von Zellen und/oder Zellbereichen im Bild der Probe oder auch von sub-zellulären Strukturen erfolgen und eine Zuordnung zwischen technischem Kontrast und Farbkontrast für unterschiedliche Bereiche der Zelle und/oder Probe mittels Computerlernens.

In der späteren Anwendung können dann für die Klassifizierung der Zellen bzw. alternativ der Zellbestandteile auch auf den nur mit technischem Kontrast aufgenommenen Bildern dann die lokal für Bildpunkte oder Bildbereiche im computergestützten automatisierten Auswertesystem bestimmten Klassifizierungsmerkmale verwendet werden, die z.B. als sogenannten Feature Vektoren oder auch Aktivitätsfelder von Neuronen vorliegen können.

Das Training kann dann als erfolgreich bezeichnet werden, wenn z.B. 80 %, bevorzugt 90 %, besonders bevorzugt 95 % der Zellstrukturen in einem Validierungsdatensatz, der nicht zum Training verwendet wurde, richtig erkannt wird.

Es erfolgt also eine Klassifizierung der Zellen und Zellbestandteilen anhand von Bildern mit technischem Kontrast und anschließend eine Bewertung der Klassifizierungsgüte in einem Validierungsschritt.

Ein vorteilhafter Verfahrensablauf zur automatisierten Erkennung, Identifikation, Klassifizierung und/oder virtuellen Annotation bzw. digitalen Anfärbung einer Zelle, von Bestandteilen einer Zelle und/oder eines biologischen Präparats umfasst z.B. die folgenden Schritte.

Zunächst wird ein 2D und/oder 3D Bild der Probe mit technischem Kontrast aufgenommen. Dann erfolgt eine Analyse und/oder Segmentierung des Bildes der Probe zum Erkennen von Zellen und/oder Zellbereichen im Bild der Probe und/oder auch von sub-zellulären Strukturen. Weiter erfolgt eine digitale Einfärbung des Bildes der Probe aufgrund der vorliegenden Informationen zur 2D und/oder 3D Struktur der Zelle bzw. des Zellbereichs und/oder von Sub-zellulären Strukturen auf Basis einer vorbestimmten Farbtabelle für die unterschiedlichen Strukturen in der Zelle und/oder von Zellen. Bei der Farbtabelle handelt es sich z.B. um eine künstliche Farbtabelle und/oder um eine gelernte Farbtabelle. Weiter erfolgt die Ausgabe eines gefärbten Bildes für einen Beobachter und/oder eine Speicherung des digital eingefärbten Bildes in einer Datenbank. Weiter schließt sich vorteilhafterweise eine Befundung des Bildes bzw. der Bilder zu einer Probe z.B. eines Patienten oder eines Gewebebereichs eines Patienten für alle interessierenden Zellen oder Gewebebereiche oder Zellbereiche aufgrund der eingefärbten Bilder an. Weiter wird vorteilhafterweise ein Gesamtbefund für den Patienten erstellt.

Die 3D und/oder 2D Bilder der Probe werden dabei z.B. jeweils mittels eines automatischen Analyzer zum Analysieren einer medizinischen Probe aufgenommen, der insbesondere auch das Analysieren von Zellen in der Probe ermöglicht. Der erfindungsgemäße Analyzer wird anhand der beigefügten Zeichnung noch einmal durch ein konkretes Ausführungsbeispiel näher erläutert. Das gezeigte Beispiel stellt eine bevorzugte Ausführungsform der Erfindung dar. Es zeigt:
- FIG 1: einen automatischen Analyzer zum Analysieren von Zellen in einer Probe,
- FIG 2: den Zellkern (11) eines Lymphozyten (12) angefärbt mit DAPI (395 nm),
- FIG 3: den Zellkern (11) eines Lymphozyten (12) angefärbt mit DAPI (395nm) sowie die Plasmamembranen (14) von Erythrozyten (13), angefärbt mit PE-VIO 770 (647 nm),
- FIG 4: die Plasmamembran (14) eines neutrophilen Granulozyten (15) angefärbt mit einem Oberflächenmarker (488nm), Zellkern (11) angefärbt mit DAPI,
- FIG 5: die Plasmamembran (14) eines Lymphozyten (12) angefärbt mit einem Oberflächenmarker (488nm), Zellkern (11) angefärbt mit DAPI,
- FIG 6: ein Peroxisomen (16) eine neutrophilen Granulozyten (15) gefärbt mit einem Peroxisomenmarker (488nm),
- FIG 7: die Membran (17) eines Leukozyten (18) angefärbt mit Concanavalin A.

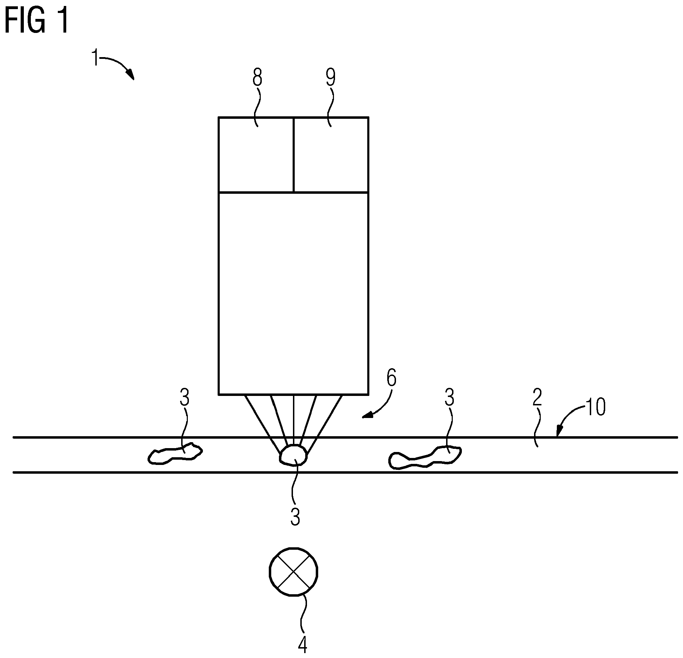

Der in FIG 1 gezeigte automatische Analyzer zum Analysieren von Zellen in einer Probe umfasst ein optisches Mikroskop (1) umfassend eine Lichtquelle (4) zum Beleuchten einer Probe (2) und eine Sammellinse zum Sammeln und Fokussieren von Lichtstrahlen (6), die von der beleuchteten Probe (2) ausgehen. Bei der Probe (2) handelt es sich um eine Blutprobe, die Blutzellen (3) enthält. Die Probe (2) befindet sich in einer mikrofluidischen Durchflusszelle (10). Weiter umfasst das Mikroskop (1) eine Lichtfeldkamera (8) umfassend ein digitales Aufzeichnungsgerät, das Aufzeichnungsgerät umfassend einen CCD Chip oder einen CMOS Chip, zum Aufnehmen des im Mikroskop (1) abgebildeten Lichtfelds. Weiter umfasst das Mikroskop (1) eine weitere Kamera zum Aufnehmen eines Bildes in der Objektebene, die als hochauflösende 3-Chip Farbkamera (9) ausgeführt ist. Die laterale Auflösung der Farbkamera (9) beträgt die vierfache effektive laterale Auflösung der Lichtfeldkamera (8). Der Fokus der Farbkamera (9) ist auf einen mittleren Bereich des Messbereichs der Lichtfeldkamera (8) eingestellt, z.B. auf eine virtual depth im Bereich von 3 bis 5. Die inkohärente Beleuchtung Sigma am Mikroskop (1) beträgt 1,0. Bei dem Mikroskop (1) handelt es sich um ein Mikroskop, das auch als Differential-Interferenz-Kontrast (DIC) Mikroskop (1) betrieben werden kann.

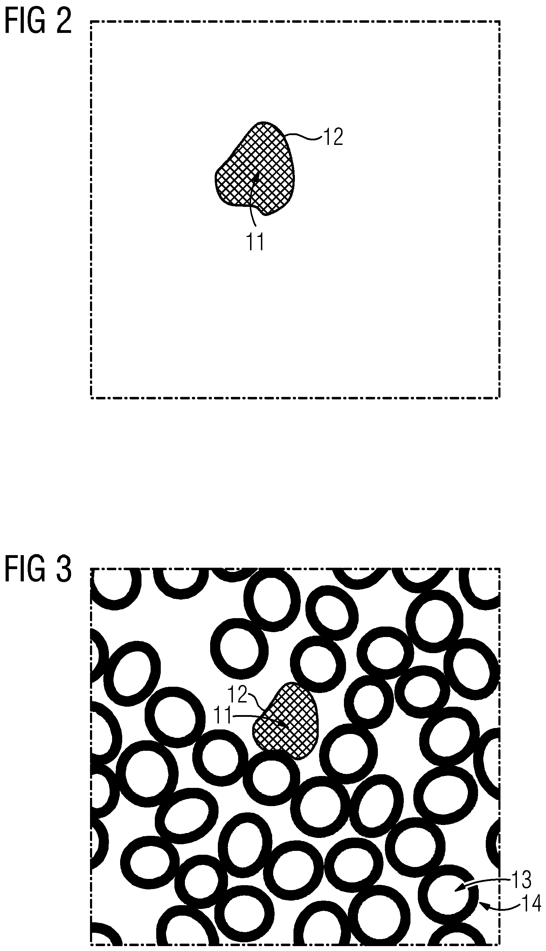

FIG 2 zeigt den Zellkern (11) eines Lymphozyten (12) angefärbt mit DAPI, das Objekt wurde mit Licht der Wellenlänge 395 nm angeregt.

FIG 3 zeigt den Zellkern (11) eines Lymphozyten (12) angefärbt mit DAPI (395nm) sowie eine Mehrzahl von Erythrozyten (13) Plasmamembranen (14) angefärbt mit PE-VIO 770, das Objekt wurde mit Licht der Wellenlänge 395 nm und 647 nm angeregt.

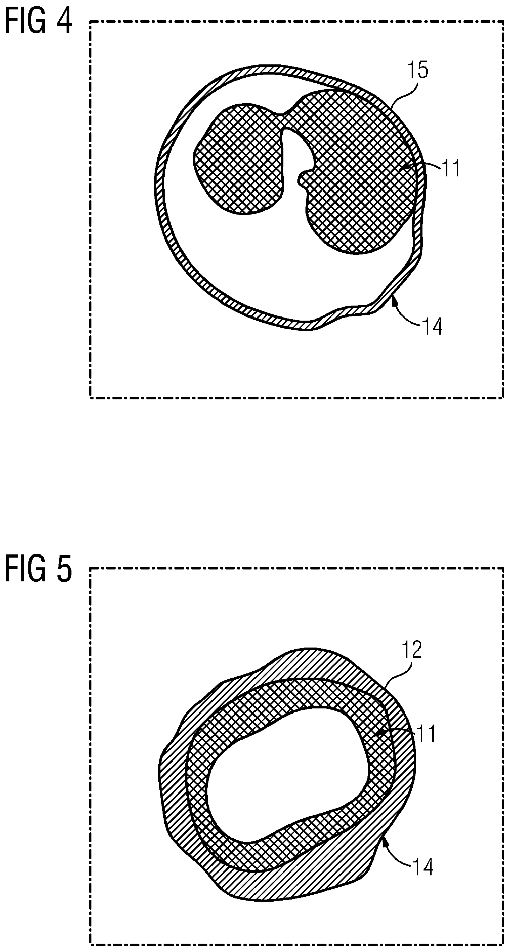

FIG 4 zeit die Plasmamembran (14) eines neutrophilen Granulozyten (15) angefärbt mit einem Oberflächenmarker mit Anregungswellenlänge 488 nm sowie den Zellkern (11), angefärbt mit DAPI. Das Objekt wurde mit Licht der Wellenlänge 395 nm und 488 nm angeregt.

FIG 5 zeigt die Plasmamembran (14) eines Lymphozyten (12) angefärbt mit einem Oberflächenmarker mit Anregungswellenlänge 488 nm sowie den Zellkern (11), angefärbt mit DAPI. Das Objekt wurde mit Licht der Wellenlänge 395 nm und 488 nm angeregt.

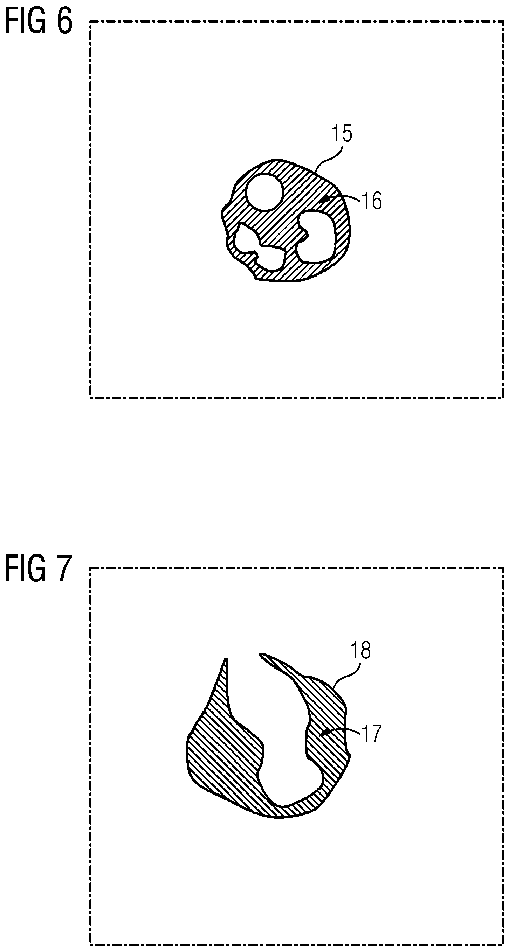

FIG 6 zeigt ein Peroxisomen (16) eines neutrophilen Granulozyten (15) gefärbt mit einem Peroxisomenmarker mit Anregungswellenlänge 488 nm. Das Objekt wurde mit Licht der Wellenlänge 488 nm angeregt.

FIG 7 zeigt die Membran (17) eines Leukozyten (18) angefärbt mit Concanavalin A.

### Bezugszeichenliste

- 1: Mikroskop
- 2: Probe
- 3: Blutzelle
- 4: Lichtquelle
- 6: Lichtstrahlen
- 8: Lichtfeldkamera
- 9: Farbkamera
- 10: Durchflusszelle
- 11: Zellkern
- 12: Lymphozyt
- 13: Erythrozyt
- 14: Plasmamembran
- 15: Neutrophiler Granulozyt
- 16: Peroxisomen
- 17: Membran
- 18: Leukozyt

## Patentansprüche

1. Verfahren zum Trainieren eines Verfahrens zur automatisierten Erkennung, Identifikation, Klassifizierung und/oder virtuellen Annotation einer Zelle, von Bestandteilen einer Zelle und/oder eines biologischen Präparats, das Verfahren umfassend die Schritte
a) Einfärbung der Zelle, der Bestandteile der Zelle und/oder des biologischen Präparats mittels Durchführung eines Färbeprotokolls, wobei das Färbeprotokoll eine selektive Färbung von vorbestimmten Merkmalen der Zelle und/oder des biologischen Präparats umfasst, wobei bevorzugt die vorbestimmten Merkmale zelluläre und/oder sub-zelluläre Strukturen umfassen, und wobei die Färbung eine selektiv schaltbare Färbung umfasst,
b) Ermitteln von zweidimensionalen und/oder dreidimensionalen Informationen der Zelle und/oder des biologischen Präparats mittels eines ersten Analyzers zum Analysieren einer biologischen Probe, der Analyzer umfassend eine Vorrichtung zur Ermittlung der zweidimensionalen und/oder dreidimensionalen Informationen der Zelle und/oder des biologischen Präparats umfassend einen Farbkontrast, wobei der Farbkontrast sensitiv ist auf die Färbung des Färbeprotokolls und wobei das Ermitteln der Informationen für wenigstens zwei unterschiedliche Schaltzustände der selektiv schaltbaren Färbung erfolgt,
c) Erstellung und Speicherung einer Zuordnung zwischen den mit den unterschiedlichen Schaltzustände der selektiv schaltbaren Färbung ermittelten Informationen.

2. Verfahren nach Anspruch 1, wobei vor Einfärbung der Zelle, der Bestandteile der Zelle und/oder des biologischen Präparats in Schritt a) eine Ermittlung von zweidimensionalen und/oder dreidimensionalen Informationen der Zelle, der Bestandteile der Zelle und/oder des biologischen Präparats ohne Färbung mittels eines zweiten Analyzers zum Analysieren einer biologischen Probe erfolgt, der Analyzer umfassend eine Vorrichtung zur Ermittlung der zweidimensionalen und/oder dreidimensionalen Informationen der Zelle, der Bestandteile der Zelle und/oder des biologischen Präparats mit zellulären und/oder sub-zellulären Strukturen, die bevorzugt technische Kontrastierungsverfahren zur Gewinnung der zweidimensionalen und/oder dreidimensionalen Information anwendet, und wobei weiter eine Erstellung und Speicherung einer Zuordnung zwischen den vor der Anfärbung und nach der Anfärbung ermittelten zweidimensionalen und/oder dreidimensionalen Informationen der Zelle, der Bestandteile der Zelle und/oder des biologischen Präparats erfolgt.

3. Verfahren nach Anspruch 2, wobei es sich bei dem ersten Analyzer und dem zweiten Analyzer um das gleiche Gerät handelt und wobei der Analyzer bevorzugt jeweils in unterschiedlichen Messkonfigurationen betrieben wird.

4. Verfahren nach einem der vorhergehenden Ansprüche, wobei die Färbung in einem der Schaltzustände keinen oder nur einen möglichst geringen Einfluss auf das Ermitteln von zweidimensionalen und/oder dreidimensionalen Informationen der Zelle, der Bestandteile der Zelle und/oder des biologischen Präparats hat und die Zelle, die Bestandteile der Zelle und/oder das Präparat in diesem Schaltzustand besonders bevorzugt ungefärbt erscheint.

5. Verfahren nach einem der vorhergehenden Ansprüche, das Verfahren zusätzlich umfassend eine weitere Einfärbung der Zelle, der Bestandteile der Zelle und/oder des biologischen Präparats mittels Durchführung eines weiteren Färbeprotokolls, wobei das weitere Färbeprotokoll eine statische Anfärbung der Zelle, der Bestandteile der Zelle und/oder des biologischen Präparats umfasst und wobei die weitere Einfärbung bevorzugt nach Durchführung der Schritte a), b) und c) erfolgt.

6. Verfahren nach einem der vorhergehenden Ansprüche, wobei die Färbung mindestens zwei verschiedene, unabhängig voneinander selektiv schaltbare Färbungen umfasst.

7. Verfahren nach einem der vorhergehenden Ansprüche, wobei die Färbung mittels eines externen Stimulus selektiv ein und/oder ausschaltbar ist, wobei der externe Stimulus bevorzugt eine optische, thermische, elektrische und/oder magnetische Einwirkung auf die Färbung und/oder die Probe umfasst.

8. Verfahren nach einem der vorhergehenden Ansprüche, wobei die Färbung schaltbare Markierungsmoleküle umfasst.

9. Verfahren nach einem der vorhergehenden Ansprüche, wobei die schaltbaren Markierungsmoleküle Fluorophoren umfassen.

10. Verfahren nach einem der vorhergehenden Ansprüche, wobei die markierten Bestandteile der Zelle und/oder des medizinischen Präparats Organellen sind.

11. Verfahren nach einem der vorhergehenden Ansprüche, wobei die Markierung eine eindeutige Markierung der vorbestimmten Merkmale darstellt.

12. Verfahren nach einem der vorhergehenden Ansprüche, wobei es sich bei der ermittelten zweidimensionalen und/oder dreidimensionale Information um zweidimensionale und/oder dreidimensionale Bildinformationen handelt, bevorzugt um optische Bildinformationen im Realraum.

13. Verfahren nach Anspruch 12, wobei die markierten Bestandteile mittels der Bildinformationen selektiv erkennbar sind.

14. Verfahren nach einem der Ansprüche 12 oder 13, wobei sich räumlich in der Bildinformation überlagernde Merkmale, die unterschiedlich markiert sind, getrennt darstellbar sind.

15. Verfahren nach Anspruch 14, wobei die getrennte Darstellbarkeit aufgrund unterschiedlicher Stimuli und/oder der emittierten Lichtwellenlängen der Markierungsmoleküle erfolgt.

16. Verfahren nach einem der vorhergehenden Ansprüche, wobei die selektive Schaltbarkeit mittels unterschiedlicher Anregungs- und/oder Detektionswellenlängen erfolgt, wobei die Schaltbarkeit bevorzugt optisch erfolgt.

17. Verfahren nach einem der vorhergehenden Ansprüche, wobei die Bildinformation der markierten Zellen, der Bestandteile der Zellen und/oder biologischen Präparate als Referenz für Bildinformationen mit technischem Kontrast ohne vorherige Anfärbung verwendet werden, bevorzugt zur Identifikation von Bildinformationen, die geeignet sind zur Unterscheidung verschiedener Zelltypen und/oder anderer morphologischer Eigenschaften der Probe auf Grundlage von Bildinformationen mit technischem Kontrast ohne vorherige Anfärbung der Zellen, der Bestandteile der Zellen und/oder der biologischen Präparate.

18. Verfahren zur virtuellen Annotation einer Zelle, von Bestandteilen einer Zelle und/oder eines biologischen Präparats, das Verfahren umfassend die Schritte
aa) Ermitteln von zweidimensionalen und/oder dreidimensionalen Informationen der Zelle, von Bestandteilen der Zelle und/oder des biologischen Präparats mittels eines Analyzers zum Analysieren einer biologischen Probe, der Analyzer umfassend eine Vorrichtung zur Ermittlung der zweidimensionalen und/oder dreidimensionalen Informationen der Zelle, der Bestandteile der Zelle und/oder des biologischen Präparats,
bb) virtuelle Annotation der Zelle, der Bestandteile der Zelle und/oder des biologischen Präparats entsprechend einer vorbestimmten Zuordnung zwischen den zweidimensionalen und/oder dreidimensionalen Informationen der Zelle, der Bestandteile der Zelle und/oder des biologischen Präparats und einer Färbung einer entsprechenden Zelle, von Bestandteilen einer Zelle und/oder eines biologischen Präparats und/oder zellulärer und/oder sub-zellulären Strukturen der Zelle, der Bestandteile der Zelle und/oder des biologischen Präparats mittels eines Färbeprotokolls,
cc) Repräsentierung und/oder Speicherung einer Darstellung der virtuell annotierten Zelle, der Bestandteile der Zelle und/oder des biologischen Präparats,
wobei die Zuordnung in Schritt bb) mittels eines Verfahrens nach einem der Ansprüche 1 bis 17 bestimmt wurde.

19. Verfahren zur automatisierten Erkennung, Identifikation und/oder Klassifizierung einer Zelle, von Bestandteilen einer Zelle und/oder eines biologischen Präparats, das Verfahren umfassend die Schritte
aaa) Ermitteln von zweidimensionalen und/oder dreidimensionalen Informationen der Zelle, von Bestandteilen der Zelle und/oder des biologischen Präparats mittels eines Analyzers zum Analysieren einer biologischen Probe, der Analyzer umfassend eine Vorrichtung zur Ermittlung der zweidimensionalen und/oder dreidimensionalen Informationen der Zelle, der Bestandteile der Zelle und/oder des biologischen Präparats,
bbb) automatisierte Erkennung, Identifikation und/oder Klassifizierung der Zelle, der Bestandteile der Zelle und/oder des biologischen Präparats aufgrund einer vorbestimmten Zuordnung zwischen den zweidimensionalen und/oder dreidimensionalen Informationen der Zelle, der Bestandteile der Zelle und/oder des biologischen Präparats und einer Färbung einer entsprechenden Zelle, der Bestandteile einer Zelle und/oder eines biologischen Präparats und/oder zellulärer und/oder sub-zellulären Strukturen der Zelle, der Bestandteile der Zelle und/oder des biologischen Präparats mittels eines Färbeprotokolls, wobei die Zuordnung mittels eines Verfahrens nach einem der Ansprüche 1 bis 17 bestimmt wurde.

20. Analyzer umfassend eine Steuervorrichtung und/oder eine Auswertevorrichtung, wobei die Steuervorrichtung und/oder die Auswertevorrichtung so konfiguriert sind, dass der Analyzer ein Verfahren nach einem der Ansprüche 1 bis 19 durchführen kann.
